# EUROPÄISCHE PATENTSCHRIFT

(11) **EP 0 909 764 B1**
(45) Veröffentlichungstag und Bekanntmachung des Hinweises auf die Patenterteilung: **29.09.1999**
(21) Anmeldenummer: 98118613.3
(22) Anmeldetag: 01.10.1998
(51) Int. Cl.: C07J 71/00, A61K 31/58, C07J 41/00

(54) **11 Beta-Benzaldoxim-9 Alpha, 10 Alpha-epoxy-estr-4-en-Derivate, Verfahren zu ihrer Herstellung und diese Verbindungen enthaltende pharmazeutische Präparate**
11 Beta-Benzaldoxim-9 Alpha, 10 Alpha-epoxy-estr-4-en-derivatives, a process for their production and pharmaceutical compositions containing them
Dérivés 11 beta-benzaldoxim-9 alpha, 10 alpha-epoxy-estr-4-enes, un procédé pour leur préparation et compositions pharmaceutiques les contenant

(30) Priorität: 11.10.1997 DE 19745085
(43) Veröffentlichungstag der Anmeldung: 21.04.1999
(73) Patentinhaber: Jenapharm GmbH & Co. KG, 07745 Jena (DE)
(72) Erfinder: Schubert, Gerd, Dr., 07743 Jena (DE); Ring, Sven, 07749 Jena (DE); Kaufmann, Günter Dr., 07743 Jena (DE); Schneider, Birgitt, 07745 Jena (DE); Elger, Walter, Dr., 14195 Berlin (DE)

(56) Entgegenhaltungen:
- EP-A- 0 184 471
- EP-A- 0 245 170
- EP-A- 0 648 778
- WO-A-96/12494
- WO-A-96/19997
- DE-A- 3 307 143
- DE-A- 4 332 284
- FR-A- 2 586 021
- US-A- 4 447 424
- US-A- 4 634 695

## Beschreibung

Die Erfindung betrifft neue 9α,10α-Epoxide von Benzaldoxim-4,9-estradienen, deren Herstellung und diese Verbindungen enthaltende pharmazeutische Präparate.

9α,10α-Epoxide von 11β-substituierten Estra-4,9-dienen sind bereits bekannt. So werden durch Roussel Uclaf 9α,10α-Epoxide von 11β-(4-N,N-Di,methyl-aminophenyl)- estr-4-en-3-onen im EP 0 057 115 B1 und von 11β-(4-Alkinylphenyl)- estr-4-en-3-onen im EP 245 170 A1, durch Schering 9α,10α-Epoxide von 11β-(4-Acetylphenyl)- estr-4-en-3-onen und durch Kasch und Ponsold 9α,10α-Epoxide von 11β-(4-Methoxyphenyl-, 4-Cyanophenyl-, 4-Formylphenyl- und 4-Acetylphenyl-Derivaten) von Estr-4-en-3-onen im DD 289 542 beschrieben. Über die biologische Aktivität derartiger Verbindungen ist bisher wenig bekannt (G. Teutsch, D. Philibert. 1994. History and perspectives of anti-progestins from the chemist's point of view. Hum. Reprod. (Suppl. 1):12-31.

9α,10α-Epoxide von 11β-Benzaldoximderivaten der Estra-4,9-diene sind bisher nicht beschrieben.
Ihre biologische Wirkung ist noch nicht bekannt.

Das Hormon Progesteron greift zusammen mit Oestrogenen aktiv in cyclische monatliche Änderungen der Uterusschleimhaut ein. Es hat einen besonderen Einfluß auf den Menstruationszyklus und auf die Erhaltung der Gravidität. So wird vom menschlichen Körper der Frau nach der Ovulation ein erhöhter Progesteronspiegel sezerniert, der zu einer entscheidenden Veränderung der Uterusschleimhaut führt. Eine solche veränderte Uterusschleimhaut ist in der Lage eine befruchtete Eizelle (Blastozyste) einzunisten (Nidation des Embryos). Die weitere Erhaltung des spezifischen Uterusgewebes nach der Nidation zur Ernährung und zum Wachstum des Embryos wird in hohem Maß ebenfalls von dem Progesteronspiegel beeinflußt.

Weiterhin ist bekannt, daß Progesteron an der Steuerung von Ovulationsvorgängen beteiligt ist. In hohen Dosen appliziert oder sezerniert besitzt Progesteron antiovulatorische Eigenschaften, weil eine Hemmung der hypophysären Gonadotropinsekretion eintritt. Dies ist eine unabdingbare Voraussetzung für die Reifung des Follikels und die anschließende Ovulation.

Neben dem Progesteron selbst scheinen auch die Progesteronrezeptoren eine bedeutende Rolle bei der Steuerung von pathophysiologischen Prozessen des menschlichen Körpers eine Rolle zu spielen. Progesteronrezeptoren befinden sich, wie nachgewiesen wurde, sowohl in erkrankten Gewebe des Endometriums (Endometriose) als auch im krebsartig entarteten Gewebe des Uterus, der Brust und des Zentralnervensystems (Meningeom). Die Stimulierung der Bildung dieser Progesteronrezeptoren erfolgt durch Oestrogene. Die Rezeptoren übermitteln die Wirkung des Hormons Progesteron bei der Transkription. Es wurde jedoch nachgewiesen (Chwalisz, K. et.al., Endocrinology, 129, 317-322, 1991), daß Verbindungen, die stärker als Progesteron am Progesteronrezeptor gebunden werden, die aber selbst nicht gestagen aktiv sind (sogenannte Antigestagene), wie Mifepriston = RU 38486 ( EP 0 057 115 B1) oder Onapriston = ZK 98 299 (DE-OS-3 504 421) auch bei geringen Progesteronspiegel im Blut in der Lage sind, Einfluß auf das Transskriptionsverhalten des nicht von Progesteron besetzten Progesteronrezeptors auszuüben und das Wachstum Progesteronregulierter Gewebe zu inhibieren.

Progesteron kann die Synthese von Oestrogenrezeptoren und die Bildung weiterer eigener Rezeptoren hemmen, ohne selbst eine Affinität zum Oestrogenrezeptor zu besitzen. Aber auch Antigestagene sind in der Lage, Effekte von Oestrogenen zu modulieren, obwohl auch sie keine Affinität zum Oestrogenrezeptor auf zellulärer Ebene aufweisen und durchaus fähig sind, einen Anstieg der Oestrogenrezeptoren zu bewirken. Derartige Effekte können ausgenutzt werden, um im erkrankten Gewebe, das mit Oestrogen-und/oder Progesteronrezeptoren ausgestattet ist, einen günstigen Einfluß auf Erkrankungen des Endometriums oder von Tumorgeweben auszuüben.
Antigestagene sind bei einer Endometriose von Interesse, weil sie neben der Wirkung im Gewebe auch eine Hemmung der Ovulation hervorrufen können. Durch den antiovulatorischen Effekt der Antigestagene wird die ovarielle Hormonproduktion teilweise gehemmt und damit kann der auf diesen Teil entfallende Stimulationseffekt auf das erkrankte Gewebe reduziert werden. Durch eine Hemmung der Ovulation kann bei einer Endometriose das durch Hormoneinflüsse ständig im Umbau befindliche Endometrium zeitweilig und reversibel in einen ruhenden Zustand überführt werden. Das Gewebe von Endometrioseherden kann sich auf einen normalen Status rückbilden, die Erkrankung eingedämmt werden.
Antigestagene besitzen neben ihrer Affinität zum Progesteronrezeptor auch Affinitäten zum Glucocorticoidrezeptor. Bei der Langzeitbehandlung von Patienten, bei denen die Hemmung von Progesteronrezeptoren im Vordergrund steht, sind bei den erforderlichen therapeutischen Dosierungen antiglucocorticoide Nebenwirkungen nicht erwünscht und können zum Abbruch der Therapie führen. Es ist daher sehr wünschenswert, Verbindungen zu entwickeln, die als Antigestagene nur geringe oder keine antiglucocorticoide Wirkung aufweisen, um therapeutsche Behandlungen, die Wochen oder Monate dauern können, ohne diese Nebenwirkungen durchführen zu können.

Aufgabe der vorliegenden Erfindung ist es, 11β-Benzaldoxim- 9α,10α-epoxy-estr-4-en-Derivate der allgemeinen Formel I 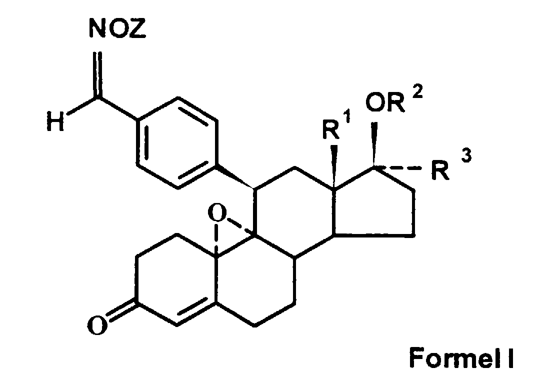 und ihre pharmazeutisch annehmbaren Salze sowie ein Verfahren zu ihrer Herstellung zur Verfügung zu stellen.
Eine weitere Aufgabe ist es, pharmazeutische Präparate, die mindestens eine Verbindung der allgemeinen Formel I oder deren pharmazeutisch annehmbaren Salze enthalten, zur Verfügung zu stellen.

In der allgemeinen Formel I 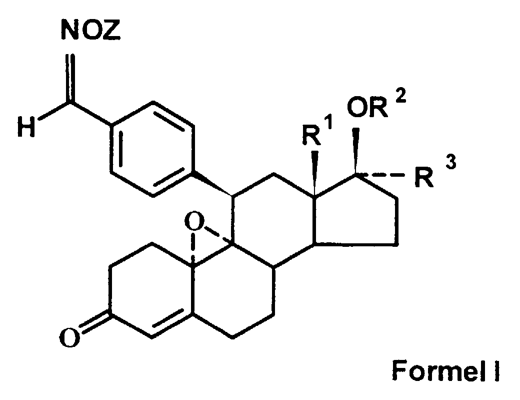 ist R¹ ein Wasserstoffatom oder ein Alkylrest mit 1-6 Kohlenstoffatomen,
steht R² für ein Wasserstoffatom, eine Alkyl-, Aryl-, Aralkyl-, oder Alkylarylgruppe mit jeweils 1-10 Kohlenstoffatomen , einen Acylrest mit 1-10 Kohlenstoffatomen oder einen Rest -CONHR⁴ oder -COOR⁴ , wobei R⁴ ein Wasserstoffatom, eine Alkyl-, Aryl-, Aralkyl- oder Alkylarylrest mit jeweils 1-10 Kohlenstoffatomen bedeutet,
steht R³ für ein Wasserstoffatom, einen Alkyl-, Aryl-, Aralkyl- oder Alkylarylgruppe mit jeweils 1-10 Kohlenstoffatomen, einen Rest -(CH₂)ₙCH₂Y, wobei n = 0, 1 oder 2 ist, Y für ein Fluor-, Chlor-, Brom-, oder lodatom für eine Cyano-, Azido- oder Rhodanogruppe, für einen Rest OR⁵ oder SR⁵ steht, wobei R⁵ ein Wasserstoffatom, ein Alkyl-, Aryl-, Aralkyl- oder Alkylarylrest mit jeweils 1-10 Kohlenstoffatomen oder ein Acylrest COR⁵ mit 1-10 Kohlenstoffatomen ist, einen Rest OR⁵, wobei R⁵ ein Wasserstoffatom, ein Alkyl-, Aryl-, Aralkyl- oder Alkylarylrest mit jeweils 1 - 10 Kohlenstoffatomen oder ein Acylrest COR⁵ mit 1-10 Kohlenstoffatomen bedeutet,
einen Rest - (CH₂)ₘ-CH=CH(CH₂)ₙ-R⁶, wobei m =0, 1, 2 oder 3 und n = 0, 1 oder 2 ist und R⁶ für ein Wasserstoffatom, einen Alkyl-, Aryl-, Aralkyl- oder Alkylarylrest mit jeweils 1-10 Kohlenstoffatomen oder eine Hydroxylgruppe, eine Alkoxy- oder Acyloxygruppe mit jeweils 1-10 Kohlenstoffatomen steht,
einen Rest - (CH₂)ₒC≡CR⁷, wobei o = 0,1 oder 2 und R⁷ für ein Wasserstoffatom, ein Fluor-, Chlor-, Brom- oder lodatom, einen Alkyl-, Aryl-, Aralkyl- oder Alkylarylrest mit jeweils 1-10 Kohlenstoffatomen oder einen Acylrest mit 1-10 Kohlenstoffatomen steht,
Z für ein Wasserstoffatom, eine Alkyl-, Aryl-, Alkylaryl- oder Arylalkylgruppe mit jeweils 1-10 Kohlenatoffatomen, einen Acylrest mit 1-10 Kohlenstoffatomen, einen Rest CONHR⁴ oder COOR⁴ steht, wobei R⁴ ein Wasserstoffatom, einen Alkyl-, Aryl-, Aralkyl- oder Alkylarylrest mit jeweils 1 - 10 Kohlenstoffatomen bedeutet, oder für ein Alkali- oder Erdalkalimetallatom, sowie deren pharmazeutisch annehmbaren Salze steht.

Am meisten bevorzugt sind
4-[9α,10α-Epoxy-17β-methoxy-17α-(methoxymethyl)-3-oxoestr-4-en-11β-yl]-benzaldehyd-1-(E)-oxim,
4-[9α,10α-Epoxy-17β-methoxy-17α-(methoxymethyl)-3-oxoestr-4-en-11β-yl]-benzaldehyd-1-(Z)-oxim,
4-[9α,10α-Epoxy-17β-hydroxy-17α-(methoxymethyl)-3-oxoestr-4-en-11β-yl]-benzaldehyd-1-(E)-oxim,
4-[9α,10α-Epoxy-17β-hydroxy-17α-(methoxymethyl)-3-oxoestr-4-en-11β-yl]-benzaldehyd-1-(Z)-oxim,
4-[9α,10α-Epoxy-17β-methoxy-17α-(methoxymethyl)-3-oxoestr-4-en-11β-yl]-benzaldehyd-1-(E)-(O-acetyl)-oxim,
4-[9α,10α-Epoxy-17β-hydroxy-17α-(methoxymethyl)-3-oxoestr-4-en-11β-yl]-benzaldehyd-1-(E)-(O-acetyl)-oxim,
4-[9α,10α-Epoxy-17β-methoxy-17α-(methoxymethyl)-3-oxoestr-4-en-11β-yl]-benzaldehyd-1-(E)-[O-(methoxy)-carbonyl]-oxim,
4-[9α,10α-Epoxy-17β-hydroxy-17α-(methoxymethyl)-3-oxoestr-4-en-11β-yl]-benzaldehyd-1-(E)-[O-(methoxy)-carbonyl]-oxim,
4-[9α,10α-Epoxy-17β-methoxy-17α-(methoxymethyl)-3-oxoestr-4-en-11β-yl]-benzaldehyd-1-(E)-[O-(ethoxy)-carbonyl]-oxim,
4-[9α,10α-Epoxy-17β-hydroxy-17α-(methoxymethyl)-2-oxoestr-4-en-11β-yl]-benzaldehyd-1-(E)-[O-(ethoxy)-carbonyl]-oxim,
4-[9α,10α-Epoxy-17β-methoxy-17α-(ethoxymethyl)-3-oxoestr-4-en-11β-yl]-benzaldehyd-1-(E)-oxim,
4-[9α,10α-Epoxy-17β-hydroxy-17α-(ethoxymethyl)-3-oxoestr-4-en-11β-yl]-benzaldehyd-1-(E)-oxim,
4-[9α,10α-Epoxy-17β-ethoxy-17α-(methoxymethyl)-3-oxoestr-4-en-11β-yl]-benzaldehyd-1-(E)-oxim,
4-[9α,10α-Epoxy-17β-methoxy-17α-(2-propoxymethyl)-3-oxoestr-4-en-11β-yl]-benzaldehyd-1-(E)-oxim,
4-[9α,10α-Epoxy-17β-hydroxy-17α-(2-propoxymethyl)-3-oxoestr-4-en-11β-yl]-benzaldehyd-1-(E)-oxim,
4-[9α,10α-Epoxy-17β-methoxy-17α-(methoxymethyl)-3-oxoestr-4-en-11β-yl]-benzaldehyd-1-(E)-[O-(phenyloxy)-carbonyl]-oxim,
4-[9α,10α-Epoxy-17β-hydroxy-17a-(methoxymethyl)-3-oxoestr-4-en-11 β-yl]-benzaldehyd-1-(E)-[O-(N-ethylamino)-carbonyl]-oxim,
4-[9α,10α-Epoxy-17β-methoxy-17α-(methoxymethyl)-3-oxoestr-4-en-11β-yl]-benzaldehyd-1-(E)-[O-(N-ethylamino)-carbonyl]-oxim,
4-[17α-Chlormethyl-9α,10α-epoxy-17β-hydroxy-3-oxoestr-4-en-11β-yl]-benzalde-hyd-1-(E)-oxim:
4-[9α,10α-Epoxy-17β-acetoxy-17α-(methoxymethyl)-3-oxoestr-4-en-11β-yl]-benzaldehyd-1-(E)-oxim.

Die Erfindung betrifft ferner ein Verfahren zur Herstellung der Verbindungen nach der allgemeinen Formel I und deren pharmazeutisch annehmbaren Salze, das dadurch gekennzeichnet ist, daß man eine Verbindung der allgemeinen Formel II, 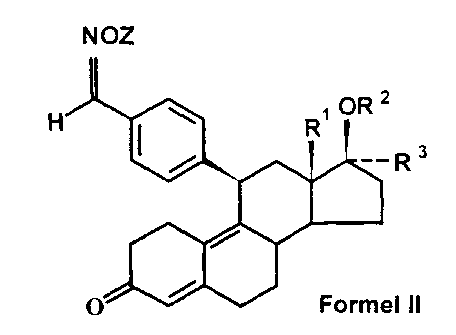 worin R¹, R², R³ und Z die vorstehend angegebene Bedeutung besitzt mit einem Oxydationsmittel wie Persäuren oder Wasserstoffperoxid in Lösungen oder Suspensionen umsetzt, die gegebenenfalls vorliegende freie 11β-Benzaldimin-gruppe zu einer anderen Verbindung der Formel (I) verestert, verethert oder in ein annehmbares Salz überführt.

Bei Wunsch nach Veresterung der freien Hydroxylgruppen (Z, R² = H) kann die Veresterung in an sich bekannter Weise mit Säureanhydriden oder Säurechloriden in Gegenwart von Basen, vorzugsweise Pyridin, in besonderen Fällen auch in Gegenwart von Dimethylaminopyridin, die Urethanbildung durch Reaktion mit Isocyanaten in inerten Lösungsmitteln oder durch Reaktion mit Carbamoylchloriden und die Veretherung mit Alkyl-oder Arylhalogeniden in Gegenwart von Basen, vorzugsweise Kalium tert.-butanolat durch geführt werden.

Die Herstellung der Ausgangsverbindungen, die auch Antigestagene sind, erfolgt nach der Offenlegungsschrift DE 4 332 283.

Gegebenenfalls wird die erhaltene erfindungsgemäße Verbindung der allgemeinen Formel 1 in ein Säureadditionssalz, vorzugsweise in ein Salz einer physiologisch verträglichen Säure überführt. Übliche physiologisch verträgliche Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere verwendbare Säuren sind beispielsweise in Fortschritte der Arzneimittelforschung, Bd.10, Seiten 224-225, Birkhäuser Verlag, Basel und Stuttgart, 1966 beschrieben. Die Nucleophilie der Säuren darf unter den Reaktionsbedingungen dabei nicht ausreichend sein, um die Epoxydgruppe zu öffnen.
Die Säureaddtionsverbindungen werden in der Regel in an sich bekannter Weise durch Mischen der freien Base oder deren Lösungen mit einer entsprechenden Säure oder deren Lösungen in einem organischen Lösungsmittel hergestellt. Ferner ist es möglich, physiologisch verträgliche wäßrige Lösungen von Säureadditionssalzen der Verbindungen der allgemeinen Formel I in wäßrigen Säurelösungen herzustellen.
Die Säureadditionsprodukte der allgemeinen Verbindung I können in an sich bekannter Weise mit Alkalien oder mit lonenaustauschern in die freie Base überführt werden.

Gegenstand der vorliegenden Erfindung sind Arzneistoffe zur oralen, rektalen, subcutanen, intravenösen oder intramuskulären Anwendung, die zusammen mit den üblichen Träger und Verdünnungsmitteln mindestens eine Verbindung der allgemeinen Formel I oder deren Säureadditionssalze als Wirkstoff enthalten.

Pharmazeutische Präparate der Erfindung werden mit den üblichen festen oder flüssigen Trägerstoffen und/oder Verdünnungsmitteln und den allgemein üblicherweise verwendeten Hilfsstoffe entsprechend der gewünschten Applikationsart in einer geeigneten Dosierung und in an sich bekannter Weise hergestellt. Bei einer bevorzugten oralen Darreichungsform werden vorzugsweise Tabletten, Filmtabletten, Dragees, Kapseln, Pillen, Pulver,Lösungen oder Suspensionen auch als Depotform zubereitet.

Daneben sind parenterale Arzneiformen wie Injektionslösungen oder aber Suppositorien in Betracht zu ziehen.
Arzneiformen als Tabletten können beispielsweise durch Mischen des Wirkstoffes mit bekannten Hilfsstoffen, wie Dextrose. Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln, die einen Depoteffekt erzielen können, wie Carboxylpolymethylen, Carboxymethylcellulose, Celluloseacetatphthalat oder Polyvinylacetat, erhalten werden. Die Tabletten können auch aus mehreren Schichten bestehen.
Analog lassen sich Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid, oder Zucker bereiten. Die Drageehülle kann dabei auch aus mehreren Schichten bestehen, wobei beispielsweise oben genannte Hilfsstoffe verwendet werden.
Die Lösungen oder Suspensionen mit dem erfindungsgemäßen Wirkstoff können zur Verbesserung des Geschmacks mit Stoffen wie Saccharin, Cyclamat oder Zucker und / oder mit Aromastoffen, wie Vanillin oder Orangeextrakt versetzt werden. Weiterhin können sie mit Suspendierhilfsstoffen wie Natriumcarboxyme-thylcellulose oder Konservierungsmitteln wie p-Hydroxybenzoesäure vermischt werden.
Die Bereitung von Kapseln kann durch Mischen des Arzneistoffes mit Trägern wie Milchzucker oder Sorbit erfolgen, die dann in die Kapseln eingebracht werden.
Die Herstellung von Suppositorien erfolgt vorzugsweise durch Mischung des Wirkstoffes mit geeigneten Trägermaterialien wie Neutralfetten oder Polyethylenglykolen oder dessen Derivaten.

Die erfindungsgemäßen 11β-Benzaldoxim-9α,10α-epoxy-estr-4-en-Derivate sind antigestagen wirkende Verbindungen, die nur mit mittlerer Affinität am Progesteron-rezeptor gebunden werden ( vgl. Tab. 1) aber überraschenderweise in vivo subcutan an der Ratte eine stärkere Wirkung als RU 486 (Mifepriston) und als die entsprechenden 4,9-Diene der Formel II aufweisen ( vgl. Tab. 2).

Es konnte keinerlei Bindung an den Estrogenrezeptor nachgewiesen werden. Danach basiert auch der in-vivo-Effekt auf der antigestagenen Wirkung der erfindungsgemäßen Verbindungen.

**Tabelle 1**

| Rezeptorbindung von ausgewählten 11β-Benzaldoxim-9α, 10α-epoxy-estr-4-en-Derivaten | | |
|---|---|---|
| Verbindung nach Beispiel | relative molare Bindungsaffinität RBA (%) zum Progesteronrezeptor Progesteron = 100 % | relative molare Bindungsaffinität RBA (%) zum Glucocorticoidrezeptor Dexamethason = 100 % |
| | | |
| 1 ( J 1102) | 88 | 12 |
| 2 (J 1160) | 49 | 18 |
| 3 ( J 1116) | 47 | 12 |
| 4 (J 1182) | 29 | |
| 5 ( J 1130) | 59 | 15 |
| 6 ( J 1180) | 31 | 8 |
| 9 ( J 1120) | 63 | 15 |
| 16 (J 1186) | 64 | 13 |
| 17 (J 1131) | 68 | 30 |
| | | |
| zum Vergleich: | | |
| RU 486 (Mifepriston) | 506 | 685 |
| J 867 | 302 | 77 |
| ZK 98299 (Onapriston) | 22 | 39 |

**Tabelle 2**

| Frühabortive Wirkung bei der Ratte nach subcutaner Applikation vom 5.-7. Graviditätstag [Applikation 0,2 ml /Tier/ Tag in Benzoylbenzoat /Rizinusöl (1+4 v/v)] | | | |
|---|---|---|---|
| Substanz | Dosis (mg/Tier/Tag) | komplette Graviditätshemmung * | |
| | | N^{#} /N | % |
| | | | |
| Vehikel | | 0/6 | 0 |
| | | | |
| Beispiel 1 (J 1102) | 3 | 5/5 | 100 |
| | 1 | 5/5 | 100 |
| | 0,3 | 0/5 | 0 |
| | | | |
| Beispiel 3 (J 1116) | 3 | 5/5 | 100 |
| | 1 | 5/5 | 100 |
| | 0,3 | 5/5 | 100 |
| | 0,1 | 2/4 | 50 |
| | | | |
| Beispiel 8 ( J 1204) | 1 | 5/5 | 100 |
| | | | |
| Beispiel 9 (J 1120) | 3 | 5/5 | 100 |
| | 1 | 5/5 | 100 |
| | 0,3 | 1/5 | 20 |
| | | | |
| RU 486 | 3 | 5/5 | 100 |
| | 1 | 1/5 | 20 |
| | 0,3 | 0/5 | 0 |
| | | | |
| J 867 | 3 | 5/5 | 100 |
| | 1 | 5/5 | 100 |
| | 0,3 | 0/5 | 0 |

| | | | |
|---|---|---|---|
| * leere Uteri N Zahl der angepaarten Weibchen N^{#} Zahl der nichtgraviden Weibchen | | | |

Die erfindungsgemäßen Verbindungen der allgemeinen Formel I sollen an den nachfolgenden Beispielen näher erläutert, jedoch nicht eingeschränkt werden.

### Beispiele

### Allgemeine Vorschrift zur Epoxidation der 9,10 Doppelbindung:

(2 mmol) 4-[17β-substituiertes-17α-substituiertes-3-oxo-estr-4-en-11β-yl]-benzalde-hyd-1-oxim-Derivat werden in 10 ml Methylenchlorid gelöst und bei 23 °C mit (3 mmol) m-Chlorperbenzoesäure versetzt und gerührt. Nach 45 Minuten wird mit wäßriger Natriumthiosulfatlösung versetzt, die Phasen werden getrennt. Die organische Phase wird mit Wasser, wäßriger Natriumbicarbonat-Lösung und Wasser gewaschen, über Na₂SO₄ getrocknet und unter Vakuum verdampft. Das anfallende Rohprodukt wird durch Umkristallisation oder durch Chromatographie und anschließende Umkristallisation gereinigt.

### Beispiel 1

### 4-[9α,10α-Epoxy-17β-methoxy-17α-(methoxymethyl)-3-oxoestr-4-en-11β-yl)-benzaldehyd-1-(E)-oxim:

Ausbeute: 74 % d.Th.
Schmp. 137 - 143 °C (Aceton/Methyl-*tert.*butylether); α_{D} = + 142 ° (CHCl₃); IR in KBr [cm⁻¹]: 1662 (C=C-C=O); UV [MeOH]: λₘₐₓ 254 nm ε = 23 700, ¹H-NMR: [CDCl₃; TMS] (δ, ppm): 0,49 (s, 3H, H-18); 3,18 (d,1H, J = 6,3 Hz, 11αH); 3,26 (s, 3H , OCH₃); 3,41 (s, 3H, OCH₃); 3,44 und 3,61 (2d, 2H, J = 10.8 Hz, 17α-CH₂OCH₃); 6,11 (s, 1H, H-4); 7,28 (d, 2H, J=7,8 Hz, H-2'); 7,52 (d, 2H, J=7,8 Hz, H-3'); 7,85 (s, 1H, OH);8,12 (s, 1H CH=N-OH).

### Beispiel 2

### 4-[90,10α-Epoxy-17β-methoxy-17α-(methoxymethyl)-3-oxoestr-4-en-11β-yl]-benzaldehyd-1-(Z)-oxim:

Schmp. 144-150 °C (Aceton/n-Hexan); α_{D} = + 141 ° (CHCl₃); IR in KBr [cm⁻¹]: 1660 (C=C-C=O); UV [MeOH]: λₘₐₓ 251 nm; ¹H-NMR: [CDCl₃] TMS] (δ, ppm): 0,49 (s, 3H, H-18); 3,26 (s, 3H , OCH₃); 3,41 (s, 3H, OCH₃); 3,45 und 3,63 (2d, 2H, J = 10.9 Hz, 17α-CH₂OCH₃); 6,11 (s, 1H, H-4); 7,33 (d, 2H, J = 8,3 Hz, H-2'); 7,88 (d, 2H, J = 8,3 Hz, H-3'); 7,33 (s, 1H CH=N-OH)

### Herstellung der Ausgangsverbindung

### 4-[17β-Methoxy-17α-(methoxymethyl)-3-oxo-estra-4,9-dien-11β-yl]benzaldehyd-1-(Z)-oxim

Die Verbindung entsteht bei der Herstellung von 4-[17β-Methoxy-17α-(methoxy-methyl)-3-oxo-estra-4,9-dien-11β-yl]-benzaldehyd-1-(E)-oxim (gemäß DE 43 32 283) und wird durch Chromatographie von dieser abgetrennt und isoliert.
Schmp. 120-138 °C (Methyl-tert.butylether); α_{D} = 217 ° (CHCl₃); IR in KBr [cm⁻¹]: 1649 (C=C-C=O); UV [MeOH]: λₘₐₓ 256 nm; ¹H-NMR: [CDCl₃; TMS] (δ, ppm): 0,54 (s, 3H, H-18); 3,26 (s, 3H , OCH₃); 3,41 (s, 3H, OCH₃); 3,42 und 3,58 (2d, 2H, J = 10.5 Hz, 17α-CH₂OCH₃); 4,39 (d, J = 7.3 Hz,H-11) 5,79 (s, 1H, H-4); 7,26 (d, 2H, J = 8,9 Hz, H-2' ); 7,88 (d, 2H, J = 8,9 Hz, H-3'); 7,33 (s, 1H CH=N-OH); 9,38 (s, 1H, OH).

### Beispiel 3

### 4-[90,10α-Epoxy-17β-hydroxy-17α-(methoxymethyl)-3-oxoestr-4-en-11β-yl]-benzaldehyd-1-(E)-oxim:

Ausbeute: 47 % d. Th.
Schmp. 145 - 155 °C Zers. (Aceton); α_{D} = + 145 ° (CHCl₃); IR in KBr [cm⁻¹] 1660 (C=C-C=O); UV [MeOH]: λₘₐₓ 255 nm ε = 24 800, ¹H-NMR: [CDCl₃; TMS] (δ, ppm): 0,48 (s, 3H, H-18); 2,85 (s, 1H, OH); 3,20 (d, 1H, J = 6,3 Hz, 11α-H); 3,42 (s, 3H, OCH₃); 3,25 und 3,63 (2d, 2H, J = 9,0 Hz, 17α-CH₂OCH₃)1 6,11 (s, 1H, H-4); 7,27 (d, 2H, J = 7,8 Hz, H-2'); 7,52 (d, 2H, J =7,8 Hz, H-3')); 7,87 (s, 1H, OH); 8,11 (s, 1H CH=N-OH).

### Beispiel 4

### 4-[9α,10α-Epoxy-17β-hydroxy-17α-(methoxymethyl)-3-oxoestr-4-en-11β-yl]-benz-aldehyd-1-(Z)-oxim:

Schmp. 145-155 °C (Aceton/n-Hexan); α_{D} = + 142 ° (CHCl₃); IR in KBr [cm⁻¹]: 1662 (C=C-C=O); UV [MeOH]: λₘₐₓ 252 nm; ¹H-NMR: [CDCl₃; TMS] (δ, ppm): 0,50 (s, 3H, H-18); 3,21 (d, 1H, J = 6.04 Hz, H-11α); 3,42 (s, 3H, OCH₃); 3,27 und 3,63 (2d, 2H, J = 9.2 Hz, 17α-CH₂OCH₃); 6,12 (s, 1H, H-4); 7,33 (d, 2H, J = 8,4 Hz, H-2' ); 7,93 (d, 2H, J = 8,4 Hz, H-3'); 7,34 (s, 1H CH=N-OH) ; 9,92 (s, 1H, OH).

### Herstellung der Ausgangsverbindung

### 4-[17β-Hydroxy-17α-(methoxymethyl)-3-oxo-estra-4,9-dien-11β-yl]benzaldehyd-1-(Z)-oxim

Die Verbindung entsteht bei der Herstellung von 4-[17β-Hydroxy-17α(meth-oxymethyl)-3-oxo-estra-4,9-dien-11β-yl]-benzaldehyd-1 -(E)-oxim (gemäß DE 43 32 283) und wird durch Chromatografie von dieser abgetrennt und isoliert.
Schmp. 135-146 °C (Aceton); α_{D} = + 192 ° (CHCl₃); IR in KBr [cm⁻¹]: 1650 (C=C-C=O); UV [MeOH]: λₘₐₓ 257 nm; λₘₐₓ 302 nm ¹H-NMR: [CDCl₃; TMS] (δ, ppm): 0,54 (s, 3H, H-18); 3,42 (s, 3H, OCH₃); 3,23 und 3,57 (2d, 2H, J = 9.1 Hz, 17α-CH₂OCH₃), 4,41 (d, J = 7.2 Hz,H-11) 5,79 (s, 1H, H-4); 7,26 (d, 2H, J = 8,4 Hz, H-2'); 7,86 (d, 2H, J = 8,4 Hz, H-3'); 7,33 (s, 1H CH=N-OH) ; 8,56 (s, 1H, OH).

### Beispiel 5

### 4-[9α,10α-Epoxy-17β-methoxy-17α-(methoxymethyl)-3-oxo-estr-4-en-11β-yl]-benzaldehyd-1-(E)-[O-(acetyl)]-oxim

Ausbeute: 65 % d.Th.; Schmp. 132- 136 °C Zers. (Essigester); α_{D} = + 158 ° (CHCl₃); IR in KBr [cm⁻¹]: 1660 (C=C-C=O), 1704 (OAc); UV [MeOH]: λₘₐₓ 262 nm ε = 26 100; ¹H-NMR: [CDCl₃; TMS] (δ, ppm): 0,47 (s, 3H, H-18); 2,23 (s, 3H, COCH₃); 3,21 (d, 1H, J = 5,7 Hz, H-11α); 3,25 (s, 3H , OCH₃); 3,42 (s, 3H, OCH₃); 3,44 und 3,61 (2d, 2H, J = 10.8 Hz, 17α-CH₂OCH₃); 6,11 (s, 1H, H-4); 7,33 (d, 2H J= 8,4 Hz, H-2'); 7,69 (d, 2H, J = 8,4 Hz, H-3'); 8,33 (s, 1H CH=N).

### Beispiel 6

### 4-[9α,10α-Epoxy-17β-hydroxy-17α-(methoxymethyl)-3-oxoestr-4-en-11β-yl]-benzaldehyd-1-(E)-(O-acetyl)-oxim,

Schmp. 125-135 °C (Essigester); α_{D} = + 125 ° (CHCl₃); IR in KBr [cm' ¹]: 1663 (C=C-C=O), 1768 (OAc); UV [MeOH]: λₘₐₓ 262 nm; ¹H-NMR: [CDCl₃; TMS] (δ, ppm): 0,47 (s, 3H, H-18); 2,23 (s, 3H, OCOCH₃); 3,21 (d, 1H, J = 4.5 Hz, H-11α); 3,24 und 3,62 (2d, 2H, J = 9.1 Hz, 17α-CH₂OCH₃), 3,42 (s, 3H, OCH₃); 6,11 (s, 1H, H-4); 7.33 (d. 2H, J = 8,0 Hz, H-2'); 7,70 (d. 2H, J = 8.0 Hz, H-3'); 8,34 (s, 1H CH=N).

### Herstellung der Ausgangsverbindung:

### 4-[17β-Hydroxy-17α-(methoxymethyl)-3-oxo-estra-4,9-dien-11β-yl]benzaldehyd-1-(E)-(O-acetyl)-oxim

2 mmol 4-[17β-hydroxy-17α-(methoxymethyl)-3-oxo-estra-4,9-dien-11β-yl]-benzalde-hyd-1-(E)-oxim werden in 7 ml Acetanhydrid / Pyridin (1:1, v:v) innerhalb von 5 Stunden bei Raumtemperatur acetyliert. Es wird in Eiswasser eingegossen, der Niederschlag wird abgesaugt.
Ausbeute: 85 % d.Th.; Schmp. 115 °C Zers. (Essigester); α_{D} = + 209 ° (CHCl₃); IR in KBr [cm⁻¹]: 1655 (C=C-C=O), 1768 (OAc); 3519 (OH); UV [MeOH]: λₘₐₓ 272 nm ε = 27 900, λₘₐₓ 297 nm ε = 26 000; ¹H-NMR: [CDCl₃; TMS] (δ, ppm): 0,52 (s, 3H, H-18); 2,23 (s, 3H, OCOCH₃); 3,22 und 3,56 (2d, 2H, J = 9.0 Hz, 17α-CH₂OCH₃); 3,41 (s, 3H, OCH₃); 4,41 (d, 1H, J = 7.2 Hz, H-11α); 5,79 (s, 1H, H-4); 7,25 ((d, 2H, J = 6,6 Hz, H-2'); 7,64 (d, 4H, J = 6.6 Hz, H-3'); 8,32 (s, 1H CH=N).

### Beispiel 7

### 4-[9α,10α-Epoxy-17β-methoxy-17α-(methoxymethyl)-3-oxoestr-4-en-11β-yl]-benzaldehyd-1-(E)-[O-(methoxy)-carbonyl]-oxim:

Schmp. 164 -175 °C (Methyl-tert.butylether/Hexan)); α_{D} = + 156 ° (CHCl₃); IR in KBr [cm⁻¹]: 1670 (C=C-C=O), 1789 (OAc); UV [MeOH]: λₘₐₓ 259 nm ε = 27 060;
¹H-NMR: [CDCl₃; TMS] (δ, ppm): 0,47 (s, 3H, H-18); 3,21 (d, 1H, J = 6.0 Hz, H-11α);
3,25 (s, 3H, OCH₃); 3,42 (s, 3H, OCH₃); 3,44 und 3,62 (2d, 2H, J = 10.8 Hz, 17α-CH₂OCH₃); 6,11 (s, 1H, H-4); 7,33 (d, 2H J= 8,4 Hz, H-2'); 7,68 (d, 2H, J = 8,4 Hz, H-3'); 8,32 (s, 1H CH=N).

### Herstellung der Ausgangsverbindung:

### 4-[17β-Methoxy-17α-(methoxymethyl)-3-oxo-estra-4,9-dien-11β-yl]benzaldehyd-1-(E)-[O-methoxy)-carbonyl]-oxim

450 mg 4-[17β-Methoxy-17α-(methoxymethyl)-3-oxo-estra-4,9-dien-11β-yl]-benz-aldehyd-(E)-oxim werden in 10 ml Pyridin mit 0,5 ml Chlorameisensäuremethylester unter Eiskühlung innerhalb von 2 h umgesetzt. Es wird in Eiswasser eingerührt, abgesaugt und getrocknet. Der farblose Niederschlag wird durch Chromatographie an Kieselgel gereinigt und aus Methyl-tert.butylether umkristallisiert.
Ausbeute : 70 % d. Th.; Schmp.:110 -123 °C (Zers.) α_{D} = + 171 ° (CHCl₃); IR in KBr [cm⁻¹]: 1655 (C=C-C=O), 1769 (OAc); UV [MeOH]: λₘₐₓ 269 nm ε = 21 700 λₘₐₓ 297 nm ε = 21 300; ¹H-NMR: [CDCl₃; TMS] (δ, ppm): 0,51 (s, 3H, H-18); 3,25 (s, 3 H, OCH₃); 3,40 und 3,55 (2d, 2H, J = 9.0 Hz, 17α-CH₂OCH₃); 3,41 (s, 3H, OCH₃); 3,92 (s, 3H, COOCH₃) 4,40 (d, 1H, J = 7.2 Hz, H-11α); 5,78 (s, 1H, H-4); 7,26 (d, 2H, J = 8,1 Hz., H-2'); 7,63 (d, 4H, J = 8,1 Hz, H-3'); 8,31 (s, 1H CH=NOR).

### Beispiel 8

### 4-[9α,10α-Epoxy-17β-hydroxy-17α-(methoxymethyl)-3-oxoestr-4-en11β-yl]-benzaldehyd-1-(E)-[O-(methoxy)-carbonyl)-oxim

Schmp. 168 - 175 °C (Aceton); α_{D} = + 144 ° (CHCl₃); IR in KBr [cm⁻¹] 1663 (C=C-C=O); 1750, 1770, 1789 (MeO-(C=O)-O-N=CH); UV [MeOH]: λₘₐₓ 215nm ε = 23 978, log ε = 4.38; λₘₐₓ 259 nm ε = 29 810, log ε = 4.47; ¹H-NMR : [CDCl₃; TMS] (δ, ppm): 0,47 (s, 3H, H-18); 3,24 (d, 1H, J = 6,0 Hz, H-11α); 3,42 (s, 3H, OCH₃); 3,25 und 3,62 (2d, 2H, J = 9,3 Hz, 17α-CH₂HOCH₃); 6,11 (s, 1H, H-4); 7,33 (d, 2H, J = 8,1 Hz, H-2'); 7,69 (d, 2H, J = 8,4 Hz, H-3'); 8,33 (s, 1H CH=N-OR).

### Herstellung der Ausgangsverbindung:

### 4-[17β-Hydroxy-17α-(methoxymethyl)-3-oxoestra-4,9-dien-11β-yl]-benzaldehyd-1-(E)-[O-methoxy)-carbonyl]-oxim

560 mg 4-[17β-Hydroxy-17α-(methoxymethyl)-3-oxo-estra-4,9-dien-11β-yl]-benz-aldehyd-1-(E)-oxim werden in 10 ml Pyridin mit 0,5 ml Chlorameisen-säuremethylester unter Eiskühlung innerhalb von 2 h umgesetzt. Es wird in Eiswasser eingerührt, abgesaugt und getrocknet. Der blaßgelbe Niederschlag wird durch Chromatographie an Kieselgel gereinigt und aus Aceton/Methyl-tert.butylether umkristallisiert.
Ausbeute : 61 % d. Th.; Schmp.: 158-163 °C (Zers.); α_{D} = + 203° (CHCl₃); IR in KBr [cm⁻¹]: 1648 (C=C-C=O), 1747, 1781[O-(C=O)-OCH₃]; UV [MeOH]: λₘₐₓ 269 nm ε = 25 700 λₘₐₓ 297 nm ε = 24 200; ¹H-NMR: [CDCl₃; TMS] (δ, ppm): 0,51 (s, 3H, H-18); 3,21 und 3,56 (2d, 2H, J = 7,5 Hz, 17α-CH₂OCH₃); 3,41 (s, 3H, OCH₃); 3,93 (s, 3H, COOCH₃) 4,41 (d, 1H, J = 7.2 Hz, H-11α); 5,79 (s, 1H, H-4); 7,26 (d, 2H, J = 7,2 Hz, H-2'); 7,64 (d, 4H, J = 8,4 Hz, H-3'); 8,32 (s, 1H CH =NOR).

### Beispiel 9

### 4-[9α,10α-Epoxy-17β-methoxy-17α-(methoxymethyl)-3-oxoestr-4-en-11β-yl)-benzaldehyd-1-(E)-[O-(ethoxy)-carbonyl]-oxim,

Ausbeute: 75 % d.Th.; Schmp. 171 - 173 °C Zers. (Aceton); α_{D} = + 160 ° (CHCl₃); IR in KBr [cm⁻¹]: 1670 (C=C-C=O), 1783 [O(C=O)OCH₃]; UV [MeOH]: λₘₐₓ 259 nm ε = 26 600, logε = 4.42; ¹H-NMR: [CDCl₃; TMS] (δ, ppm): 0,47 (s, 3H, H-18); 1,39 (t, 3H, J = 7,2 Hz, CH₃CH₂O); 3,21 (d, 1H, J = 6,0 Hz, H-11α); 3,25 (s, 3H , OCH₃); 3,42 (s, 3H, OCH₃); 3,45 und 3,62 (2d, 2H, J = 10.8 Hz, 17α-CH₂OCH₃); 4,35 (m, 2H, CH₂); 6.11 (s, 1H, H-4); 7,33 (d, 2H, J = 8,4 Hz, H-2'); 7,68 (d, 2H, J = 8,4 Hz, H-3'); 8,32 (s, 1H CH=N).

### Beispiel 10

### 4-[9α,10α-Epoxy-17β-hydroxy-17α-(methoxymethyl)-3-oxoestr-4-en-11β-yl]-benz-aldehyd-(E)-[O-(ethoxy)-carbonyl]-oxim:

Ausbeute: 64 % d.Th.; Schmp. 172 - 177 °C (Aceton); α_{D} = + 147 ° (CHCl₃); IR in KBr [cm⁻¹]: 1672 (C=C-C=O), 1774 [O-(C=O)-OC₂H₅]; UV [MeOH]: λₘₐₓ 260 nm ε = 28 880; ¹H-NMR: [CDCl₃; TMS] (δ, ppm): 0,47 (s, 3H, H-18); 1,39 (t, 3H, J = 7,2 Hz, CH₃CH₂O); 2,69 (s, 1H, OH); 3,20 (d, 1H, J = 6,0 Hz, H-11α); 3,42 (s, 3H, OCH₃); 3,23 und 3,62 (2d, 2H, J = 10.8 Hz, 17α-CH₂OCH₃), 4,36 (q, 2H, CH₂); 6,11 (s, 1H, H-4); 7,33 (d, 2H, J = 8,4 Hz, H-2'); 7,69 (d, 2H, J = 8,4 Hz, H-3'); 8,32 (s, 1H CH=N).

### Herstellung der Ausgangsverbindung

890 mg 4-[17β-Hydroxy-17α-(methoxymethyl)-3-oxo-estra-4,9-dien-11β-yl]-benzal-dehyd-1-(E)-oxim werden in 15 ml Pyridin mit 1ml Chlorameisensäureethylester unter Eiskühlung innerhalb von 1 h umgesetzt. Es wird in Eiswasser eingerührt, abgesaugt und getrocknet. Der blaßgelbe Niederschlag wird aus Methylenchlorid/Methyl-tert.butylether umkristallisiert.
Ausbeute : 72 % d. Th.; Schmp.: 151 -171 °C (Zers.); α_{D} = + 201° (CHCl₃); IR in KBr [cm⁻¹]: 1655 (C=C-C=O), 1742, 1758 [O-(C=O)-OC₂H₅]; UV [MeOH]: λₘₐₓ 270 nm ε = = 25 890; λₘₐₓ 297 nm ε = 24 270; ¹H-NMR: [CDCl₃; TMS] (δ, ppm): 0,52 (s, 3H, H-18); 1,38 (t, 3H, J = 7,2 Hz, CH₂CH₃); 2,57 (s, 1H, OH); 3,21 und 3,56 (2d, 2H, J = 9,0 Hz, 17α-CH₂OCH₃); 3,41 (s, 3H, CH₂OCH₃); 4, 34 (q, 2H, OCH₂CH₃); 4,37 (d, 1H, J = 7.2 Hz, H-11α); 5,79 (s, 1H, H-4); 7,25 (d, 2H, J = 7,2 Hz, H-2'); 7,64 (d, 4H, J = 8,4 Hz, H-3'); 8,31 (s, 1H CH =NOR).

### Beispiel 11

### 4-[9α,10α-Epoxy-17β-methoxy-17α-(ethoxymethyl)-3-oxoestr-4-en-11β-yl]-benzaldehyd-1-(E)-oxim:

Ausbeute: 60 % d.Th.; Schmp. 192 - 196 °C (Methyl-tert.butylether / Hexan); α_{D} = + 135 ° (CHCl₃); IR in KBr [cm⁻¹]: 1666 (C=C-C=O); UV [MeOH]: λₘₐₓ 209 nm ε = 21200, ¹H-NMR: [CDCl₃; TMS] (δ, ppm): 0,50 (s, 3H, H-18); 1,11 (t, 3H, J = 6,9 Hz, CH₂CH₃); 3,19 (d, 1H, J = 6,0 Hz, 11αH); 3,40 (s, 3H, OCH₃); 3,45 und 3,64 (2d, 2H, J = 10,8 Hz, 17α-CH₂OCH₃); 3,49 (m, 2H, CH₂CH₃); 6,11 (s, 1H, H-4); 7,28 (d, 2H, J = 8,4 Hz, H-2'); 7,53 (d, 4H, J = 8,4 Hz, H-3'); 7,55 (s, 1H, OH); 8,11 (s, 1H CH=N-OH).

### Beispiel 12

### 4-[9α,10α-Epoxy-17β-ethoxy-17α-(methoxymethyl)-3-oxoestr-4-en-11β-yl]-benzaldehyd-(E)-oxim,

Ausbeute: 52 % d.Th.; Schmp. 192 - 196 °C (Methyl-tert.butylether/Hexan); α_{D} = + 165 ° (CHCl₃); IR in KBr [cm⁻¹]: 1674 (C=C-C=O), UV [MeOH]: λₘₐₓ 254 nm ε = 27 200; ¹H-NMR: [CDCl₃; TMS] (δ, ppm): 0,50 (s, 3H, H-18); 1,11 (t, 3H, J = 6,9 Hz, CH₂CH₃); 3,19 (d, 1H, J = 6,0 Hz, H-11α); 3,40 (s, 3H, OCH₃); 3,45 und 3,64 (2d, 2H, J = 10.8 Hz, 17α-CH₂OCH₃); 3,49 (m, 2H, CH₂CH₃); 6,11 (s, 1H, H-4); 7,28 (d, 2H, J = 8,4 Hz, H-2'); 7,53 (d, 2H, J = 8,4 Hz, H-3'); 8,11 (s, 1H CH=N).

### Beispiel 13

### 4-[9α,10α-Epoxy-17β-methoxy-17α-(2-propoxymethyl)-3-oxoestr-4-en-11β-yl]-benzaldehyd-1-(E)-oxim:

Ausbeute: 74 % d.Th.; Schmp. 121 - 130 °C (Aceton/ Hexan); α_{D} = + 122 ° (CHCl₃), IR in KBr [cm⁻¹]: 1682 (C=C-C=O); UV [MeOH]: λₘₐₓ 254 nm ε = 23 150; ¹H-NMR: [CDCl₃; TMS] (δ, ppm): 0,49 (s, 3H, H-18); 1,22 [m, 6H, ΣJ = 9,9 Hz, (CH₃)₂ CHO); 3,19 (d, 1H, J = 5,7 Hz, H-11α); 3,27 (s, 3H, OCH₃); 3,47 und 3,66 [2d, 2H, J = 9 Hz, (CH₃)₂-CH-O-CH₂]; 3,6 (m, 1H, -O-CH-C); (6,11 (s, 1H, H-4); 7,28 (d, 2H, J = 8,4 Hz, H-2'); 7,52 (d, 2H, J = 8,4 Hz, H-3'); 7,78 (s, 1H, NOH); 8,11 (s, 1H CH=N).

### Herstellung der Ausgangsverbindung

Stufe A
   Zu 3 g 3,3 -Dimethoxy-11β-[4-(dimethoxymethyl)phenyl]17(S)-spirooxiran-estr-9-en-5α-ol in 25 ml DMSO werden 2,3 g Natriumisopropylat zugegeben und bei 80 °C gerührt. Die Lösung wird in 1,5 l Eiswasser eingegossen und abgesaugt. Der Niederschlag wird in Essigester gelöst und getrocknet. Das Lösungsmittel wird unter Vakuum verdampft. Man erhält 2,99 g 3,3-Dimethoxy-11β-[4-(dimethoxymethyl)-phenyl]17α-(2-propoxymethyl)-estr-9-en-5α,17β-diol als braunen Schaum, der direkt in die nächste Stufe eingesetzt wird.
Stufe B
   2,99 g 3,3-Dimethoxy-11β-[4-(dimethoxymethyl)-phenyl]17α-(2-propoxymethyl)-estr-9-en-5α,17β-diol werden in 100ml Toluol gelöst und mit 8 g Kalium tert.butanolat unter Argonschutz bei Raumtemperatur gerührt. Nach 10 Minuten werden tropfenweise 5 ml Methyliodid zugegeben. Nach 1,5 h wird 150 ml Wasser und 100 ml Essigester zugegeben und die Phasen werden getrennt. Die organische Phase wird neutral gewaschen über Na₂SO₄ getrocknet und unter Vakuum eingedampft. Man erhält 3,5 g 3,3-Dimethoxy-11β-[4-(dimethoxymethyl)-phenyl]17β-methoxy-17α-(2-propoxymethyl)-estr-9-en-5α-ol als braunen Schaum, der direkt in die nächste Stufe eingesetzt wird.
Stufe C
   3,5 g 3,3-Dimethoxy-11β-[4-(dimethoxymethyl)-phenyl]17β-methoxy-17α-(2-prop-oxymethyl)-estr-9-en-5α-ol werden in 50 ml Methyl-tert.butylether gelöst. Bei Raum-temperatur fügt man unter Argonschutz 5 ml Wasser und 250 mg p-To-luolsulfonsäure zu. Man neutralisiert mit wäßriger Bicarbonat-Lösung, wäscht die organische Phase mit Wasser, trocknet sie über Na₂SO₄, filtriert ab und engt unter Vakuum ein. Man erhält 1,95 eine hellbraunen Schaumes, der durch Chro-matogrphie an Kieselgel mit einem Toluol/Aceton-Gradienten gereinigt wird.
   Ausbeute: 1,3 g (52 % d.Th.) 4-[17β-Methoxy-17α-(2-propoxymethyl)-3-oxoestra-4,9-dien-11β-yl]benzaldehyd-1.
   Schmp. 167 - 177 °C (Methyl-tert.butylether/Hexan); α_{D} = + 173 ° (CHCl₃); IR in KBr
   [cm⁻¹]: 1660 (C=C-C=O); 1694 (CHO); UV [MeOH]: λₘₐₓ 263 nm ε = 19 420, λₘₐₓ 299 nm ε = 21 840; ¹H-NMR: [CDCl₃; TMS] (δ, ppm): 0,50 (s, 3H, H-18); 1,22 [t, 6H, J = 5,8 Hz, (CH₃)₂ CH-O-); 2,96 (s, 1H, OCH₃); 3,44 und 3,58 [2d, 2H, J =10,4 und 10,8 Hz, (CH₃)₂-CH-O-CH₂]; 3,59 (m,2H, CH₂-CH); 4,42 (d, 1H, J = 7,3 Hz, H-11α); 5,79 (s, 1H, H-4); 7,37 (d, 2H, J = 8,1 Hz, H-2'); 7,79 (d, 2H, J = 8,3 Hz, H-3'); 9,96 (s, 1H CH=O).
Stufe D
   910 mg 4-[17β-Methoxy-17α-(2-propoxymethyl)-3-oxoestra-4,9-dien-11β-yl]benzal-dehyd-1 werden in 30 ml Pyridin mit 152 mg Hydroxylaminhydrochlorid bei Raumtemperatur umgesetzt. Nach 3 h wird in Eiswasser eingegossen, der Niederschlag wird abgesaugt, getrocknet und chromatographisch gereinigt. Man erhält 690 mg 4-[17β-Methoxy-17α-(2-propoxymethyl)-3-oxoestra-4,9-dien-11β-yl]benzaldehyd-1-(E)-oxim, die aus Aceton /Hexan umkristallisiert werden.
   Schmp. 143 Zers. °C (Aceton/Hexan); α_{D} = + 199 ° (CHCl₃); IR in KBr [cm⁻¹]: 1633 (C=C-C=O); UV [MeOH]: λₘₐₓ 264 nm ε = 23 080, λₘₐₓ 299 nm ε = 22 670; ¹H-NMR: [CDCl₃; TMS] (δ, ppm): 0,54 (s, 3H, H-18); 1,22 [t, 6H, J = 6,0 Hz, (CH₃)₂ CH-O-); 3,26 (s, 1H, OCH₃); 3,43 und 3,62 [2d, 2H, J =10,5 und 10,8 Hz, (CH₃)₂-CH-O-CH₂]; 3,59 (m,2H, CH₂-CH); 4,37 (d, 1H, J = 6,9 Hz, H-11α); 5,79 (s, 1H, H-4); 7,21 (d, 2H, J = 8,1 Hz, H-2'); 7,48 (d, 2H, J = 8,4 Hz, H-3'); 7,99 (s, 1H, OH); 8,1 (s, 1H CH=NH).

### Beispiel 14

### 4-[9α,10α-Epoxy-17β-hydroxy-17α-(2-propoxymethyl)-3-oxoestr-4-en-11β-yl)-benzaldehyd-1-(E)-oxim:

Ausbeute: 59 % d.Th.; Schmp. 199 - 223 °C (Aceton); α_{D} = + 147 ° (CHCl₃); IR in KBr [cm⁻¹]: 1660 (C=C-C=O); UV [MeOH]: λₘₐₓ 255 nm ε = 27 220; ¹H-NMR: [CDCl₃; TMS] (δ, ppm): 0,49 (s, 3H, H-18); 1,21 [m, 6H, ΣJ = 9,9 Hz, (CH₃)₂ CH-O-); 3,0 (s, 1H, OH); 3,20 (d, 1H, J = 5,7 Hz, H-11α); 3,26 und 3,63 [2d, 2H, J = 9,1 Hz, (CH₃)₂-CH-O-CH₂]; 3,63 [sep. -OCH-(CH₃)₂]; (6,11 (s, 1H, H-4); 7,28 (d, 2H, J = 8,4 Hz, H-2'); 7,52 (d, 2H, J = 8,4 Hz, H-3'); 7,99 (s, 1H, NOH); 8,11 (s, 1H CH=N).

### Herstellung der Ausgangsverbindung

Stufe A
   2,99 g 3,3-Dimethoxy-11β-[4-(dimethoxymethyl)phenyl]17α-(2-propoxymethyl)-estr-9-en-5α,17β-diol-ol werden als Rohprodukt in 30 ml Aceton gelöst, mit 3 ml Wasser und 300 mg p-Toluolsulfonsäure versetzt und 2,5 h bei Raumtemperatur gerührt. Anschließend wird Essigester zugesetzt, mit Wasser neutral gewaschen getrocknet und das Lösungsmittel unter Vakuum verdampft. Man erhält 2,45 g als Schaum. Nach chromatographischer Reinigung an Kieselgel werden 1,16 g 4-[17β -Hydroxy-17α-(2-propoxymethyl)-3-oxoestra-4,9-dien-11β-yl]benzaldehyd-1 isoliert.
   Schmp. 132 - 142 °C (Methyl-tert.butylether/Hexan); α_{D} = + 186 ° (CHCl₃); IR in KBr [cm⁻¹]: 1660 (C=C-C=O); 1705 (CHO); UV [MeOH]: λₘₐₓ 264 nm ε = 19 720, λₘₐₓ 299 nm ε = 22 300; ¹H-NMR: [CDCl₃; TMS] (δ, ppm): 0,51 (s, 3H, H-18); 1,21 [m, 6H, ΣJ = 9,9 Hz, (CH₃)₂ CHO); 2,84 (s, 1H, OH); 3,21 und 3,64 [2d, 2H, J = 9 Hz, (CH₃)₂-CH-O-CH₂]; 4,41 (d, 1H, J = 5,7 Hz, H-11α); 5,80 (s, 1H, H-4); 7,38 (d, 2H, J = 8,1 Hz, H-2'); 7,81 (d, 2H, J = 8,4 Hz, H-3'); 9,97 (s, 1H CH=O).
Stufe B
   500 mg 4-[17β-Hydroxy-17α-(2-propoxymethyl)-3-oxoestra-4,9-dien-11yl]benzalde-hyd-1 werden in 20 ml Pyridin mit 70 mg Hydroxylaminhydrochlorid innerhalb von 3 h bei Raumtemperatur umgesetzt. Man rührt in Eiswasser ein, saugt den Niederschlag ab und trocknet an der Luft. Das Rohprodukt wird durch Chromatographie gereinigt und aus Aceton umkristallisiert. Man erhält 312 mg 4-[17β-Hydroxy-17α-(2-propoxymethyl)-3-oxoestra-4,9-dien-11β-yl]-benzaldehyd-1(E)-oxim Schmp. 196 - 203 °C (Ether); α_{D} = + 215 ° (CHCl₃); IR in KBr [cm⁻¹]: 1666 (C=C-C=O); UV [MeOH]: λₘₐₓ 264 nm ε = 22 340, λₘₐₓ 299 nm ε = 22 670; ¹H-NMR: [CDCl₃; TMS] (δ, ppm): 0,52 (s, 3H, H-18); 1,21 [m, 6H, ΣJ = 9,9 Hz, (CH₃)₂ CHO); 3,02 (s, 1H, OH); 3,22 und 3,62 [2d, 2H, J = 9 Hz, (CH₃)₂-CH-O-CH₂]; 4,39 (d, 1H, J = 6,6 Hz, H-11α); 5,79 (s, 1H, H-4); 7,19 (d, 2H, J = 8,1 Hz, H-2'); 7,47 (d, 2H, J = 8,1 Hz, H-3'); 8,07 (s, 1H, OH); 8,10 (s, 1H CH=N).

### Beispiel 15

### 4-[9α,10α-Epoxy-17β-methoxy-17α-(methoxymethyl)-3-oxoestr-4-en-11β-yl]-benzaldehyd-1-(E)-[O-(phenyloxy)-carbonyl]-oxim:

Schmp. 145 - 150 °C (EtOH/Hexan); α_{D} = + 137 ° (CHCl₃); IR in KBr [cm⁻¹]: 1665 (C=C-C=O), 1796, 1808 (O-(C=O)-OPh); UV [MeOH]: λₘₐₓ 261 nm ε = 30 500; ¹H-NMR: [CDCl₃; TMS] (δ, ppm): 0,47 (s, 3H, H-18); 3,22 (d, 1H, J = 6,0 Hz, H-11α); 3,26 (s, 3H , OCH₃); 3,42 (s, 3H, OCH₃); 3,45 und 3,62 (2d, 2H, J = 10.8 Hz, 17α-CH₂OCH₃); 6,11 (s, 1H, H-4); 7,25 -7,46 (m, 5H, Aromat) 7,34 (d, 2H, J = 8,4 Hz, H-2'); 7,71 (d, 2H, J = 8,4 Hz, H-3'); 8,42 (s, 1H CH=N).

### Herstellung der Ausgangsverbindung:

1,79 g 4-[17β-Methoxy-17α-(methoxymethyl)-3-oxoestra-4,9-dien-11β-yl]-benzalde-hyd-1-(E)-oxim werden in 22 ml Pyridin mit 1,5 ml Chlorameisensäurephenylester unter Eiskühlung innerhalb von 2 h umgesetzt. Es wird in Eiswasser eingerührt, abgesaugt und getrocknet. Der blaßgelbe Niederschlag wird durch Umkristallisation aus Aceton/Hexan gereinigt. Ausbeute : 51 % d. Th.; Schmp.: 101-106 °C (Ace-ton/Hexan); α_{D} = + 179° (CHCl₃); IR in KBr [cm⁻¹] 1655 (C=C-C=O); 1728 ,1791 (PhO-C(O)-O-N=CH);UV [MeOH]: λₘₐₓ 270nm ε = 25 506, λₘₐₓ 297 nm ε = 22 803; ¹H-NMR : [CDCl₃; TMS] (δ, ppm): 0,52 (s, 3H, H-18); 3,25 (s, 3H , OCH₃); 3,41 (s, 3H, OCH₃); 3,42 und 3,57 (2d, 2H, J = 10.8 Hz, 17α-CH₂OCH₃); 4,41 (d,1 H, H-11α); 5,79 (s, 1H, H-4); 7,25- 7,46 (m, 5H, Aromat); 7,41 (d, 2H, J = 7,5 Hz, H-2'); 7,66 (d, 2H, J = 8,4 Hz, H-3'); 8,41 (s, 1H, CH=N-OR).

### Beispiel 16

### 4-[9α,10α-Epoxy-17β-hydroxy-17α-(methoxymethyl)-3-oxo-estr-4-en-11β-yl]-benzaldehyd-1-(E)-[O-(N-ethylamino)-carbonyl]-oxim:

Schmp. 192-198 °C (Aceton); α_{D} = + 154 ° (CHCl₃); IR in KBr [cm⁻¹] 1662(C=C-C=O); 1732, 1748 [C₂H₅NH-(C=O)-O-N=CH]; UV [MeOH]: λₘₐₓ 262 nm; ¹H-NMR: [CDCl₃; TMS] (δ, ppm): 0,48 (s, 3H, H-18); 1,24 (t, 3H, J = 7,2 Hz, NHCH₂CH₃); 3,42 (s, 3H, OCH₃); 3,24 und 3,62 (2d, 2H, J = 9.1 Hz, 17α-CH₂OCH₃); 6,11 (s, 1H, H-4); 6,26 (t, 1H, NHEt); 7,35 (d, 2H, J = 8,3 Hz, H-2'); 7,64 (d, 2H, J = 8,3 Hz, H-3'); 8,31 (s, 1H CH=N-OR).

### Herstellung der Ausgangsverbindung

### 4-[17β-Hydroxy-17α-(methoxymethyl)-3-oxo-estra-4,9-dien-11β-yl]-benzaldehyd-1-(E)-[O-(N-ethylamino)carbonyl]-oxim

2 mmol 4-[17β-Hydroxy-17α-(methoxymethyl)-3-oxo-estra-4,9-dien-11β-yl]-benzal-dehyd-1 -(E)-oxim werden in 10 ml Toluol mit 2,4 ml Ethylisocyanat und 4,5 ml Triethylamin innerhalb von 24 Stunden bei Raumtemperatur umgesetzt. Nach Zugabe von wäßrigen Ammoniak werden die Phasen getrennt, die organische Phase mit Ammoniumchlorid und Wasser gewaschen, getrocknet und unter Vakuum verdampft. Nach chromatographischer Reinigung und Umkristallisation werden farblose Kristalle erhalten.
Schmp. 184 -188°C (Aceton/Hexan). α_{D} = + 224° (CHCl₃); IR in KBr [cm⁻¹] 1655 (C=C-C=O); 1733, 1748 (C₂H₅NH-C=O-O-N=CH); UV [MeOH]: λₘₐₓ 272 nm ε = 28 600, λₘₐₓ 297 nm ε = 25 900 ; ¹H-NMR: [CDCl₃; TMS] (δ, ppm): 0,53 (s, 3H, H-18); 1,24 (t, 3H, J = 7,2 Hz, NHCH₂CH₃); 3,42 (s, 3H, OCH₃); 3,22 und 3,57 (2d, 2H, J = 9,1 Hz, 17α-CH₂OCH₃); 6,11 (s, 1H, H-4); 6,26 (t, 1H, NHEt); 7,35 (d, 2H, J = 8,4 Hz, H-2'); 7,64 (d, 2H, J = 8,4 Hz, H-3'); 8,30 (s, 1H CH=N-OR).

### Beispiel 17

### 4-[9α, 10α-Epoxy-17β-methoxy-17α-(methoxymethyl)-3-oxo-estr-4-en-11β-yl]-benzaldehyd-1-(E)-[O-(N-ethylamino)carbonyl)]-oxim

Ausbeute : 698 mg (57 % d.Th.); Schmp. 149 - 152 °C (MeOH); α_{D} = + 153 ° (CHCl₃); IR in KBr [cm⁻¹] 1672 (C=C-C=O); 1726 ,1749 (C₂H₅NH-C=O-O-N=CH); UV [MeOH]: λₘₐₓ 262 nm ε = 27 200; ¹H-NMR: [CDCl₃; TMS] (δ, ppm): 0,48 (s, 3H, H-18); 1,24 (t, 3H, J = 7,2 Hz, NHCH₂CH₃), 3,22 (d, 1H, J = 6,4 Hz, H-11α); 3,26 (s, 3H , OCH₃); 3,42 (s, 3H, OCH₃); 3,49 und 3,60 (2d, 2H, J = 10.8 Hz, 17α-CH₂OCH₃); 6,11 (s, 1H, H-4); 6,22 (t, 1H, NHEt); 7,34 (d, 2H, J = 8,4 Hz, H-2'); 7,63 (d, 2H, J = 8,4 Hz; H-3') 8,31 (s, 1H CH=N-OR).

### Beispiel 18

### 4-[17α-Chlormethyl-9α,10α-epoxy-17β-hydroxy--3-oxoestr-4-en-11β-yl]-benzalde-hyd-1-(E)-oxim:

Schmp. 218 -221 °C (Methyl-tert.butylether); α_{D} = + 157 ° (CHCl₃); IR in KBr [cm⁻¹] 1660 (C=C-C=O); UV [MeOH]: λₘₐₓ 209 nm ε = 24 080, log ε = 4.38; λₘₐₓ 254 nm
ε = 26 680; ¹H-NMR [CDCl₃; TMS] (δ, ppm): 0,57 (s, 3H, H-18); 3,22 (d, 1H, J = 6,0 Hz, H-11α); 3,70 und 3,98 (2d, 2H, J = 10.8 Hz, 17α-CH₂OCH₃); 6,12 (s, 1H, H-4); 7,27 (d, 2H, J = 7,8 Hz, H-2'); 7,54 (d, 2H, J = 8,1 Hz, H-3'); 7,60 (s, 1H, NOH); 8,11 (s, 1H CH=N-OH).

### Beispiel 19

### Messung der Rezeptor-Bindungsaffinität

Die Rezeptor-Bindungsaffinität wurde bestimmt durch competitive Bindung eines spezifisch bindenden ³H-markierten Hormons (Tracer) und der zu testenden Verbindung an Rezeptoren im Cytosol aus tierischen Target-Organen. Dabei wurden Rezeptorsättigung und Reaktionsgleichgewicht angestrebt. Folgende Inkubationsbe-dingungen wurden gewählt:
Progesteron-Rezeptor: Uterus-Cytosol des Estradiol-geprimten Kaninchens, aufbe-wahrt bei -30° C. Puffer für Homogenisation und Inkubation: TED-Puffer 20 mM Tris/HCI, p_{H}= 7,4; 1mM Ethylendiamintetraacetat, 2 mM Dithiothreitol) mit 250mM Saccharose. Tracer: ³H-ORG 2058, 5nM; Referenzsubstanz: Progesteron.
Glucocorticoid-Rezeptor: Thymus-Cytosol der adrenalectomierten Ratte. Thymi aufbewahrt bei -30 °C. Puffer: TED. Tracer: ³H-Dexamethason, 20nM. Referenzsub-stanz: Dexamethason.
Estrogen-Rezeptor: Uterus-Cytosol des unreifen Kaninchens, aufbe-wahrt bei -30 °C. Puffer:TED mit 250 mM Saccharose. Tracer: ³H-Estradiol, 3nM. Referenzsubstanz: 17β-Estradiol.

Nach einer Inkubation von Rezeptorfraktion, Tracer und Competitor für 18 h bei 0-4°C erfolgte die Trennung von gebundenem und freiem Steroid durch Einmischen von Aktivkohle/Dextran (1 % / 0,1 %), Abzentrifugieren und Messung der rezeptorgebundenen ³H-Aktivität im Überstand.

Aus der Messung in Konzentrationsreihen wurden die IC₅₀ für die Referenzsubstanz und für die zu testende Verbindung ermittelt und als Quotient beider Werte (x 100 %) die relative molare Bindungsaffinität bestimmt.
Nach einer Inkubationsdauer von 18 Stunden bei 0-4 °C erfolgte die Trennung von gebundenen und freiem Steroid durch Einmischen von Aktivkohle/Dextran / 1%/0,1 %), abzentrifugieren und Messung der gebundenen ³H-Aktivität im Überstand.
Aus der Messung in Konzentrationsreihen wurden die IC₅₀ für die zu testende Substanz und für die Referenzverbindung ermittelt und als Quotient beider Werte (x 100 %) die relative molare Bindungsaffinität bestimmt.

### Beispiel 20

### Hemmung der frühen Gravidität bei der Ratte

Rattenweibchen wurde im Stadium Prooestrus angepaart. Bei Nachweis von Spermien im Vaginalbereich am nächsten Tag wird dieser als Tag 1 (d =1) der Gravidität gesetzt. Die Behandlung der Ratten mit Testsubstanz oder Vehikel erfolgt mit 0,2 ml Benzoylbenzoat /Rizinusöl (1+4 v/v) subcutan vom Tag 5 bis Tag 7 (d5 -d7), die Autopsie erfolgt am Tag d 9. Die Rate von vollständig gehemmten Graviditäten in den einzelnen Gruppen ergibt sich aus Tabelle 2. So wurde für die Beispiele 1 (J 1102), 3 (J 1116), 8 (J 1204) und 9 (J 1120) eine um den Faktor 10 überlegene Nidationshemmung gegenüber RU 486 (Mifepriston) ermittelt.

## Patentansprüche

1. 11β-Benzaldoxim-9α,10α-epoxy-estr-4-en-Derivate der allgemeinen Formel I 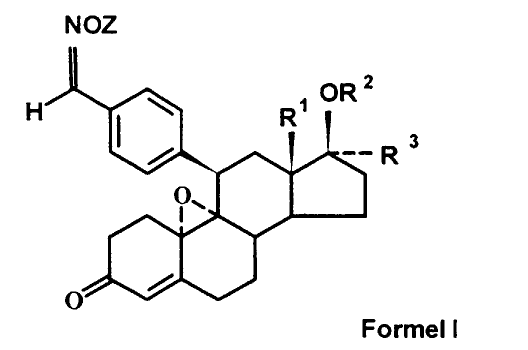 worin,
R¹ ein Wasserstoffatom oder ein Alkylrest mit 1-6 Kohlenstoffatomen ist,
R² für ein Wasserstoffatom, eine Alkyl-, Aryl-, Aralkyl-, oder Alkylarylgruppe mit jeweils 1-10 Kohlenstoffatomen, einen Acylrest mit 1-10 Kohlenstoffatomen oder einen Rest -CONHR⁴ oder -COOR⁴ steht,
wobei R⁴ ein Wasserstoffatom, einen Alkyl-, Aryl-, Aralkyl- oder Alkylarylrest mit jeweils 1-10 Kohlenstoffatomen bedeutet,
R³ für ein Wasserstoffatom, eine Alkyl-, Aryl-, Aralkyl- oder Alkylarylgruppe mit jeweils 1- 10 Kohlenstoffatomen,
einen Rest -(CH₂)ₙCH₂Y,
wobei n = 0, oder 2 ist, Y für ein Fluor-, Chlor-, Brom- oder lodatom , für eine Cyano-, Azido- oder Rhodanogruppe, für einen Rest OR⁵ oder SR⁵ steht,
wobei R⁵ ein Wasserstoffatom, einen Alkyl-, Aryl-, Aralkyl- oder Alkylarylrest mit jeweils 1-10 Kohlenstoffatomen oder ein Acylrest COR⁵ mit 1-10 Kohlenstoffatomen ist,
einen Rest OR⁵,
wobei R⁵ ein Wasserstoffatom, einen Alkyl-, Aryl-, Aralkyl- oder Alkylarylrest mit jeweils 1-10 Kohlenstoffatomen oder ein Acylrest COR⁵ mit 1-10 Kohlenstoffatomen bedeutet,
einen Rest - (CH₂)ₘ-CH=CH(CH₂)ₙ-R⁶,
wobei m =0, 1, 2 oder 3 und n = 0, 1 oder 2 ist und R⁶ für ein Wasserstoffatom, ein Alkyl-, Aryl-, Aralkyl- oder Alkylarylrest mit jeweils 1-10 Kohlenstoffatomen oder eine Hydroxylgruppe, eine Alkoxy- oder Acyloxygruppe mit jeweils 1- 10 Kohlenstoffatomen steht,
einen Rest - (CH₂)ₒC≡CR⁷,
wobei o = 0,1 1 oder 2 und R⁷ für ein Wasserstoffatom, ein Fluor-, Chlor-, Brom- oder Iodatom, ein Alkyl-, Aryl-, Aralkyl- oder Alkylarylrest mit jeweils 1-10 Kohlenstoffatomen oder ein Acylrest mit 1-10 Kohlenstoffatomen ist,
Z für ein Wasserstoffatom, eine Alkyl-, Aryl-, Alkylaryl- oder Arylalkylgruppe mit jeweils 1-10 Kohlenstoffatomen, einen Acylrest mit 1-10 Kohlenstoffatomen, einen Rest CONHR⁴ oder COOR⁴
wobei R⁴ ein Wasserstoffatom, einen Alkyl-, Aryl-, Aralkyl- oder Alkylarylrest mit jeweils 1 - 10 Kohlenstoffatomen bedeutet,
oder für ein Alkali- oder Erdalkalimetallatom steht,
sowie deren pharmazeutisch annehmbaren Salze.

2. Verbindungen nach Anspruch 1,
dadurch gekennzeichnet, daß
R¹ ein Wasserstoffatom, eine Methyl- oder Ethylgruppe ist.

3. Verbindungen nach den Ansprüchen 1 und 2,
dadurch gekennzeichnet, daß
R² für eine Alkyl- oder Arylgruppe mit jeweils 1-6 Kohlenstoffatomen, einen Acylrest mit 1-6 Kohlenstoffatomen oder einen Rest -CONHR⁴ oder -COOR⁴ steht,
wobei R⁴ ein Alkyl- oder Arylrest mit jeweils 1-6 Kohlenstoffatomen bedeutet.

4. Verbindungen nach den Ansprüchen 1 bis 3,
dadurch gekennzeichnet, daß
R³ für eine Alkylgruppe mit 1 - 6 Kohlenstoffatomen steht.

5. Verbindungen nach den Ansprüchen 1 bis 3,
dadurch gekennzeichnet, daß
R³ für einen Rest OR⁵ steht,
wobei R⁵ ein Alkyl- oder Arylrest mit jeweils 1 - 6 Kohlenstoffatomen oder ein Acylrest COR⁵ mit 1- 6 Kohlenstoffatomen ist.

6. Verbindungen nach den Ansprüchen 1 - 3,
dadurch gekennzeichnet, daß
R³ einen Rest - (CH₂)ₒC≡CR⁷ darstellt,
wobei o = 0,1 oder 2 und R⁷ ein Alkyl- oder ein Acylrest mit jeweils 1-6 Kohlenstoffatomen ist.

7. Verbindungen nach Anspruch 1,
nämlich
4-[9α,10α-Epoxy-17β-methoxy-17α-(methoxymethyl)-3-oxoestr-4-en-11β-yl]-benzaldehyd-1-(E)-oxim,
4-[9α,10α-Epoxy-17β-methoxy-17α-(methoxymethyl)-3-oxoestr-4-en-11β-yl]-benzaldehyd-1-(Z)-oxim,
4-[9α,10α-Epoxy-17β-hydroxy-17α-(methoxymethyl)-3-oxoestr-4-en-11β-yl]-benzaldehyd-1-(E)-oxim,
4-[9α,10α-Epoxy-17β-hydroxy-17α-(methoxymethyl)-3-oxoestr-4-en-11β-yl]-benzaldehyd-1-(Z)-oxim,
4-[9α,10α-Epoxy-17β-methoxy-17α-(methoxymethyl)-3-oxoestr-4-en-11β-yl]-benzaldehyd-1-(E)-(O-acetyl)-oxim,
4-[9α,10α-Epoxy-17β-hydroxy-17α-(methoxymethyl)-3-oxoestr-4-en-11β-yl]-benzaldehyd-1-(E)-(O-acetyl)-oxim,
4-[9α,10α-Epoxy-17β-methoxy-17α-(methoxymethyl)-3-oxoestr-4-en-1β-yl]-benzaldehyd-1-(E)-[O-(methoxy)-carbonyl]-oxim,
4-[9α,10α-Epoxy-17β-hydroxy-17α-(methoxymethyl)-3-oxoestr-4-en-11β-yl]-benzaldehyd-1-(E)-[O-(methoxy)-carbonyl]-oxim,
4-[9α,10α-Epoxy-17β-methoxy-17α-(methoxymethyl)-3-oxoestr-4-en-1β-yl]-benzaldehyd-1-(E)-[O-(ethoxy)-carbonyl]-oxim,
4-[9α,10α-Epoxy-17β-hydroxy-17α-(methoxymethyl)-3-oxoestr-4-en-11β-yl]-benzaldehyd-1-(E)-[O-(ethoxy)-carbonyl]-oxim,
4-[9α,10α-Epoxy-17β-methoxy-17α-(ethoxymethyl)-3-oxoestr-4-en-11β-yl]-benzaldehyd-1-(E)-oxim,
4-[9α,10α-Epoxy-17β-hydroxy-17α-(ethoxymethyl)-3-oxoestr-4-en-11β-yl]-benzaldehyd-1-(E)-oxim,
4-[9α,10α-Epoxy-17β-ethoxy-17α-(methoxymethyl)-3-oxoestr-4-en-11β-yl]-benzaldehyd-1-(E)-oxim,
4-[9α,10α-Epoxy-17β-methoxy-17α-(2-propoxymethyl)-3-oxoestr-4-en-11β-yl]-benzaldehyd-1-(E)-oxim,
4-[9α,10α-Epoxy-17β-hydroxy-17α-(2-propoxymethyl)-3-oxoestr-4-en-11β-yl]-benzaldehyd-1-(E)-oxim,
4-[9α,10α-Epoxy-17β-methoxy-17α-(methoxymethyl)-3-oxoestr-4-en-11β-yl]-benzaldehyd-1-(E)-[O-(phenyloxy)-carbonyl]-oxim,
4-[9α,10α-Epoxy-17β-hydroxy-17α-(methoxymethyl)-3-oxoestr-4-en-11β-yl]-benzaldehyd-1-(E)-[O-(N-ethylamino)-carbonyl]-oxim,
4-[9α,10α-Epoxy-17β-methoxy-17α-(methoxymethyl)-3-oxoestr-4-en-11β-yl]-benzaldehyd-1-(E)-[O-(N-ethylarnino)-carbonyl]-oxim,
4-[17α-Chlormethyl-9α,10α-epoxy-17β-hydroxy--3-oxoestr-4-en-11β-yl]benzaldehyd-1-(E)-oxim
4-[9α,10α-Epoxy-17β-acetoxy-17α-(methoxymethyl)-3-oxoestr-4-en-11β-yl]-benzaldehyd-1 (E)-oxim.

8. Verfahren zur Herstellung der Verbindungen nach Anspruch 1 und deren pharmazeutisch annehmbaren Salzen,
dadurch gekennzeichnet,
daß man Verbindungen der allgemeinen Formel II, 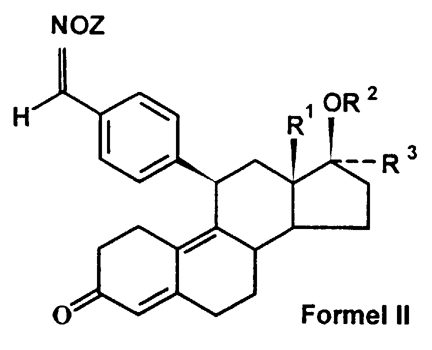 worin
R¹ ein Wasserstoffatom oder ein Alkylrest mit 1-6 Kohlenstoffatomen ist,
R² für ein Wasserstoffatom, eine Alkyl-, Aryl-, Aralkyl-, oder Alkylarylgruppe mit jeweils 1-10 Kohlenstoffatomen, einen Acylrest mit 1-10 Kohlenstoffatomen oder einen Rest -CONHR⁴ oder -COOR⁴ steht,
wobei R⁴ ein Wasserstoffatom, eine Alkyl-, Aryl-, Aralkyl- oder Alkylarylrest mit jeweils 1-10 Kohlenstoffatomen bedeutet,
R³ für ein Wasserstoffatom, einen Alkyl-, Aryl-, Aralkyl- oder Alkylarylgruppe mit jeweils 1-10 Kohlenstoffatomen, einen Rest -(CH₂)ₙCH₂Y,
wobei n = 0, 1 oder 2 ist, Y für ein Fluor-, Chlor-, Brom-, oder lodatom , für eine Cyano-, Azido- oder Rhodanogruppe,für einen Rest OR⁵ oder SR⁵
wobei R⁵ ein Wasserstoffatom, ein Alkyl-, Aryl-, Aralkyl- oder Alkylarylrest mit jeweils 1-10 Kohlenstoffatomen oder ein Acylrest COR⁵ mit 1-10 Kohlenstoffatomen ist,
einen Rest OR⁵,
wobei R⁵ ein Wasserstoffatom, ein Alkyl-, Aryl-, Aralkyl- oder Alkylarylrest mit jeweils 1-10 Kohlenstoffatomen oder ein Acylrest COR⁵ mit 1-10 Kohlenstoffatomen bedeutet,
einen Rest - (CH₂)ₘ-CH=CH(CH₂)ₙ-R⁶,
wobei m =0, 1, 2 oder 3 und n = 0, 1 oder 2 ist und R⁶ für ein Wasserstoffatom, ein Alkyl-, Aryl-, Aralkyl- oder Alkylarylrest mit jeweils 1-10 Kohlenstoffatomen oder eine Hydroxylgruppe, eine Alkoxy- oder Acyloxygruppe mit jeweils 1- 10 Kohlenstoffatomen steht,
einen Rest - (CH₂)ₒC≡CR⁷,
wobei o = 0,1 oder 2 und R⁷ für ein Wasserstoffatom, ein Fluor-, Chlor-, Brom- oder lodatom, ein Alkyl-, Aryl-, Aralkyl- oder Alkylarylrest mit jeweils 1-10 Kohlenstoffatomen oder ein Acylrest mit 1-10 Kohlenstoffatomen ist, steht,
Z für ein Wasserstoffatom, eine Alkyl-, Aryl-, Alkylaryl- oder Arylalkylgruppe mit jeweils 1-10 Kohlenstoffatomen, ein Acylrest mit 1-10 Kohlenstoffatomen, einen Rest CONHR⁴ oder COOR⁴,
wobei R⁴ ein Wasserstoffatom, eine Alkyl-, Aryl-, Aralkyl- oder Alkylarylrest mit 1 - 10 Kohlenstoffatomen bedeutet,
oder ein Alkali- oder Erdalkalimetallatom steht,
sowie deren pharmazeutisch annehmbaren Salze.
mit einem Oxydationsmittel wie Persäuren oder Wasserstoffperoxid in Lösungen oder Suspensionen umsetzt, die gegebenenfalls vorliegende freie 11 β-Benzaldimin-gruppe zu einer anderen Verbindung der Formel (I) verestert, verethert oder in ein annehmbares Salz überführt.

## Claims

1. 11β-benzaldoxime-9α, 10α-epoxy oestr-4-ene derivatives of the general formula I: 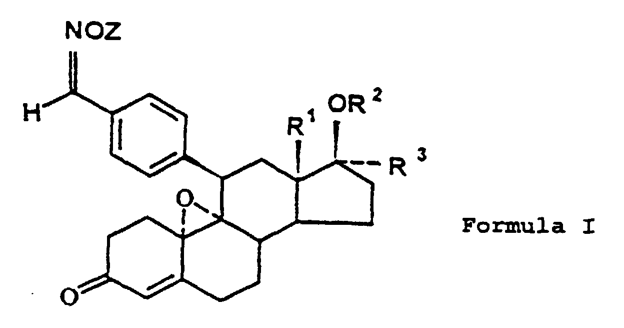 where
R¹ is a hydrogen atom or an alkyl radical containing 1-6 carbon atoms,
R² stands for a hydrogen atom, an alkyl, aryl, aralkyl or alkylaryl group containing in each case 1-10 carbon atoms, an acyl radical containing 1-10 carbon atoms or a -CONHR⁴ or -COOR⁴ radical,
where R⁴ denotes a hydrogen atom, an alkyl, aryl, aralkyl or alkylaryl radical containing in each case 1-10 carbon atoms,
R³ stands for a hydrogen atom, an alkyl, aryl, aralkyl or alkylaryl group containing in each case 1-10 carbon atoms, a -(CH₂)ₙCH₂Y radical,
where n = 0,1 or 2 and Y stands for a fluorine, chlorine, bromine or iodine atom, for a cyano, azido or rhodano group, or for a OR⁵ or SR⁵ radical,
where R⁵ is a hydrogen atom, an alkyl, aryl, aralkyl or alkylaryl radical containing in each case 1-10 carbon atoms or a COR⁵ acyl radical containing 1-10 carbon atoms,
an OR⁵ radical,
where R⁵ denotes a hydrogen atom, an alkyl, aryl, aralkyl or alkylaryl radical containing in each case 1-10 carbon atoms or a COR⁵ acyl radical containing 1-10 carbon atoms,
a -(CH₂)ₘ-CH=CH(CH₂)ₙ-R⁶ radical,
where m = 0, 1, 2 or 3 and n = 0, 1 or 2 and R⁶ stands for a hydrogen atom, an alkyl, aryl, aralkyl or alkylaryl radical containing in each case 1-10 carbon atoms or a hydroxyl group, an alkoxy or acyloxy group containing in each case 1-10 carbon atoms,
a -(CH₂)ₒC≡CR⁷ radical,
where o = 0, 1 or 2 and R⁷ stands for a hydrogen atom, a fluorine, chlorine, bromine or iodine atom, an alkyl, aryl, aralkyl or alkylaryl radical containing in each case 1-10 carbon atoms or an acyl radical containing in each case 1-10 carbon atoms,
Z stands for a hydrogen atom, an alkyl, aryl, alkylaryl or arylalkyl group containing in each case 1-10 carbon atoms, an acyl radical containing 1-10 carbon atoms, a CONHR⁴ or COOR⁴ radical,
where R⁴ denotes a hydrogen atom, an alkyl, aryl, arylalkyl or alkylaryl radical containing in each case 1-10 carbon atoms,
or for an alkali-metal or alkaline-earth-metal atom,
and also their pharmaceutically acceptable salts.

2. Compounds according to Claim 1, characterized in that
R¹ is a hydrogen atom, or a methyl or ethyl group.

3. Compounds according to Claims 1 and 2, characterized in that
R² stands for an alkyl or aryl group containing in each case 1-6 carbon atoms, an acyl radical containing 1-6 carbon atoms or a -CONHR⁴ or -COOR⁴ radical,
where R⁴ denotes an alkyl or aryl radical containing in each case 1-6 carbon atoms.

4. Compounds according to Claims 1 to 3, characterized in that
R³ stands for an alkyl group containing 1-6 carbon atoms.

5. Compounds according to Claims 1 to 3, characterized in that
R³ stands for an OR⁵ radical
where R⁵ is an alkyl or aryl radical containing in each case 1-6 carbon atoms or a COR⁵ acyl radical containing 1-6 carbon atoms.

6. Compounds according to Claims 1-3, characterized in that
R³ represents a -(CH₂)ₒC≡CR⁷ radical,
where o = 0, 1 or 2 and R⁷ is an alkyl or acyl radical containing in each case 1-6 carbon atoms.

7. Compounds according to Claim 1, namely
4-[9α,10α-epoxy-17β-methoxy-17α-(methoxymethyl)-3-oxooestr-4-ene-11β-yl]benzaldehyde-1-(E)-oxime,
4-[9α,10α-epoxy-17β-methoxy-17α-(methoxymethyl)-3-oxooestr-4-ene-11β-yl]benzaldehyde-1-(Z)-oxime,
4-[9α,10α-epoxy-17β-hydroxy-17α-(methoxymethyl)-3-oxooestr-4-ene-11β-yl]benzaldehyde-1-(E)-oxime,
4-[9α,10α-epoxy-17β-hydroxy-17α-(methoxymethyl)-3-oxooestr-4-ene-11β-yl]benzaldehyde-1-(Z)-oxime,
4-[9α,10α-epoxy-17β-methoxy-17α-(methoxymethyl)-3-oxooestr-4-ene-11β-yl]benzaldehyde-1-(E)-(O-acetyl)oxime,
4-[9α,10α-epoxy-17β-hydroxy-17α-(methoxymethyl)-3-oxooestr-4-ene-11β-yl]benzaldehyde-1-(E)-(O-acetyl)oxime,
4-[9α,10α-epoxy-17β-methoxy-17α-(methoxymethyl)-3-oxooestr-4-ene-11β-yl]benzaldehyde-1-(E)-[O-(methoxy)carbonyl]oxime,
4-[9α,10α-epoxy-17β-hydroxy-17α-(methoxymethyl)-3-oxooestr-4-ene-11β-yl]benzaldehyde-1-(E)-[O-(methoxy)carbonyl]oxime,
4-[9α,10α-epoxy-17β-methoxy-17α-(methoxymethyl)-3-oxooestr-4-ene-11β-yl]benzaldehyde-1-(E)-[O-(ethoxy)carbonyl]oxime,
4-[9α,10α-epoxy-17β-hydroxy-17α-(methoxymethyl)-3-oxooestr-4-ene-11β-yl]benzaldehyde-1-(E)-[O-(ethoxy)carbonyl]oxime,
4-[9α,10α-epoxy-17β-methoxy-17α-(ethoxymethyl)-3-oxooestr-4-ene-11β-yl]benzaldehyde-1-(E)-oxime,
4-[9α,10α-epoxy-17β-hydroxy-17α-(ethoxymethyl)-3-oxooestr-4-ene-11β-yl]benzaldehyde-1-(E)-oxime,
4-[9α,10α-epoxy-17β-ethoxy-17α-(methoxymethyl)-3-oxooestr-4-ene-11β-yl]benzaldehyde-1-(E)-oxime,
4-[9α,10α-epoxy-17β-methoxy-17α-(2-propoxymethyl)-3-oxooestr-4-ene-11β-yl]benzaldehyde-1-(E)-oxime,
4-[9α,10α-epoxy-17β-hydroxy-17α-(2-propoxymethyl)-3-oxooestr-4-ene-11β-yl]benzaldehyde-1-(E)-oxime,
4-[9α,10α-epoxy-17β-methoxy-17α-(methoxymethyl)-3-oxooestr-4-ene-11β-yl]benzaldehyde-1-(E)-[O-(phenyloxy)carbonyl]oxime,
4-[9α,10α-epoxy-17β-hydroxy-17α-(methoxymethyl)-3-oxooestr-4-ene-11β-yl]benzaldehyde-1-(E)-[O-(N-ethylamino)carbonyl]oxime,
4-[9α,10α-epoxy-17β-methoxy-17α-(methoxymethyl)-3-oxooestr-4-ene-11β-yl]benzaldehyde-1-(E)-[O-(N-ethylamino)carbonyl]oxime,
4-[17α-chloromethyl-9α,10α-epoxy-17β-hydroxy-3-oxooestr-4-ene-11β-yl]benzaldehyde-1-(E)-oxime,
4-[9α,10α-epoxy-17β-acetoxy-17α-(methoxymethyl)-3-oxooestr-4-ene-11β-yl]benzaldehyde-1-(E)-oxime.

8. Process for preparing compounds according to Claim 1 and their pharmaceutically acceptable salts, characterized in that compounds of the general formula II: 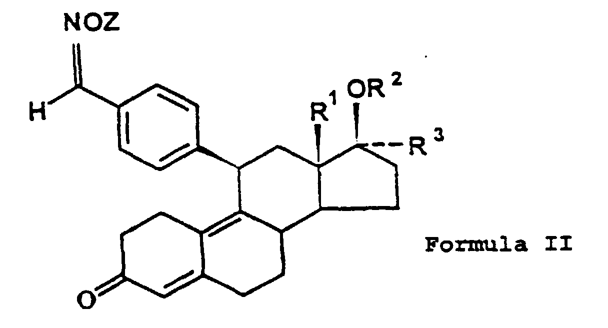 where
R¹ is a hydrogen atom of an alkyl radical containing 1-6 carbon atoms,
R² stands for a hydrogen atom, an alkyl, aryl, aralkyl or alkylaryl group containing in each case 1-10 carbon atoms, an acyl radical containing 1-10 carbon atoms or a -CONHR⁴ or -COOR⁴ radical,
where R⁴ denotes a hydrogen atom, an alkyl, aryl, aralkyl or alkylaryl radical containing in each case 1-10 carbon atoms,
R³ stands for a hydrogen atom, an alkyl, aryl, aralkyl or alkylaryl group containing in each case 1-10 carbon atoms, a -(CH₂)ₙCH₂Y radical,
where n = 0,1 or 2 and Y stands for a fluorine, chlorine, bromine or iodine atom, for a cyano, azido or rhodano group, or for a OR⁵ or SR⁵ radical,
where R⁵ is a hydrogen atom, an alkyl, aryl, aralkyl or alkylaryl radical containing in each case 1-10 carbon atoms or a COR⁵ acyl radical containing 1-10 carbon atoms,
an OR⁵ radical,
where R⁵ denotes a hydrogen atom, an alkyl, aryl, aralkyl or alkylaryl radical containing in each case 1-10 carbon atoms or a COR⁵ acyl radical containing 1-10 carbon atoms,
a -(CH₂)ₘ-CH=CH(CH₂)ₙ-R⁶ radical,
where m = 0, 1, 2 or 3 and n = 0, 1 or 2 and R⁶ stands for a hydrogen atom, an alkyl, aryl, aralkyl or alkylaryl radical containing in each case 1-10 carbon atoms or a hydroxyl group, an alkoxy or acyloxy group containing in each case 1-10 carbon atoms,
a -(CH₂)ₒC≡R⁷ radical,
where o = 0, 1 or 2 and R⁷ stands for a hydrogen atom, a fluorine, chlorine, bromine or iodine atom, an alkyl, aryl, aralkyl or alkylaryl radical containing in each case 1-10 carbon atoms or an acyl radical containing in each case 1-10 carbon atoms,
Z stands for a hydrogen atom, an alkyl, aryl, alkylaryl or arylalkyl group containing in each case 1-10 carbon atoms, an acyl radical containing 1-10 carbon atoms, a CONHR⁴ or COOR⁴ radical,
where R⁴ denotes a hydrogen atom, an alkyl, aryl, arylalkyl or alkylaryl radical containing in each case 1-10 carbon atoms,
or an alkali-metal or alkaline-earth-metal atom,
and also their pharmaceutically acceptable salts,
are reacted with an oxidizing agent such as peracids or hydrogen peroxide in solutions or suspensions to form another compound of the formula (I) and esterifies or etherifies any free 11β-benzaldimine group present or converts it into an acceptable salt.

## Revendications

1. Dérivés 11β-benzaldoxim-9α, 10α-époxy-estr-4-ènes de la formule générale 1 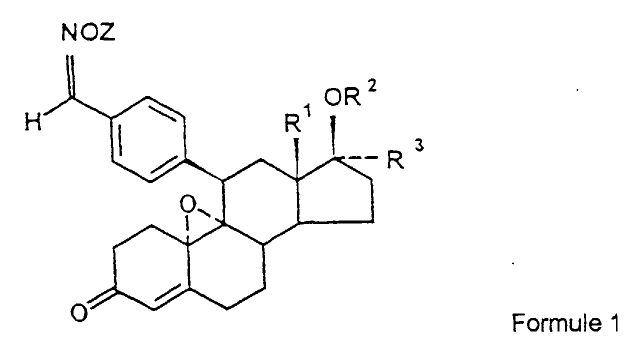 où
R¹ est un atome d'hydrogène ou un résidu alkyle avec 1-6 atomes de carbone,
R² indique un atome d'hydrogène, un groupe alkyle, aryle, aralkyle ou alkylaryle avec à chaque fois 1-10 atomes de carbone, un résidu acyle avec 1-10 atomes de carbone ou bien un résidu -CONHR⁴ ou -COOR⁴,
où R⁴ est un atome d'hydrogène, un résidu alkyle, aryle, aralkyle ou alkylaryle avec à chaque fois 1-10 atomes de carbone,
R³ indique un atome d'hydrogène, un groupe alkyle, aryle, aralkyle ou alkylaryle avec à chaque fois 1-10 atomes de carbone, un résidu-(CH₂)ₙCH₂Y,
où n = 0, 1 ou 2, Y indique un atome de fluor, de chlore, de brome, ou d'iode, un groupe cyano, azido ou rhodano, un résidu OR⁵ ou SR⁵,
où R⁵ est un atome d'hydrogène, un résidu alkyle, aryle, aralkyle ou alkylaryle, avec à chaque fois 1-10 atomes de carbone ou bien un résidu acyle COR⁵ avec 1-10 atomes de carbone,
un résidu OR⁵,
où R⁵ indique un atome d'hydrogène, un résidu alkyle, aryle, aralkyle ou alkylaryle, avec à chaque fois 1-10 atomes de carbone ou bien un résidu acyle COR⁵ avec 1-10 atomes de carbone,
un résidu - (CH₂)ₘ-CH=CH(CH₂)ₙ-R⁶,
où m = 0, 1, 2 ou 3 et n = 0, 1 ou 2 et R⁸ indique un atome d'hydrogène, un résidu alkyle, aryle, aralkyle ou alkylaryle, avec à chaque fois 1-10 atomes de carbone ou un groupe hydroxyle, un groupe alcoxy ou acyloxy avec à chaque fois 1-10 atomes de carbone,
un résidu -(CH₂)ₒC≡CR⁷,
où o = 0, 1 ou 2 et R⁷ indique un atome d'hydrogène, un atome de fluor, de chlore, de brome ou d'iode, un résidu alkyle, aryle, aralkyle ou alkylaryle avec à chaque fois 1-10 atmes de carbone ou un résidu acyle avec 1-10 atomes de carbone,
Z indique un atome d'hydrogène, un groupe alkyle, aryle, alkylaryle ou arylalkyle avec à chaque fois 1-10 atomes de carbone, un résidu acyle avec 1-10 atomes de carbone, un résidu CONHR⁴ ou COOR⁴,
où R⁴ signifie un atome d'hydrogène, un résidu alkyle, aryle, aralkyle ou alkylaryle avec à chaque fois 1-10 atomes de carbone,
ou bien indique un atome d'un métal alcalin ou alcalino-terreux,
ainsi que leurs sels pharmaceutiquement acceptables.

2. Composés selon la revedication 1,
caractérisés en ce que
R¹ est un atome d'hydrogène, un groupe méthyle ou éthyle.

3. Composés selon les revendications 1 et 2,
caractérisés en ce que
R² indique un groupe alkyle ou aryle avec à chaque fois 1-6 atomes de carbone, un résidu acyle avec 1-6 atomes de carbone ou un résidu -CONHR⁴ou -COOR⁴,
où R⁴ signifie un résidu alkyle ou aryle avec à chaque fois 1-6 atomes de carbone.

4. Composés selon les revendications 1 à 3,
caractérisés en ce que
R³ indique un groupe alkyle avec 1-6 atomes de carbone.

5. Composés selon les revendications 1 à 3,
caractérisés en ce que
R³ indique un résidu OR⁵,
où R⁵ est un résidu alkyle ou aryle avec à chaque fois 1-6 atomes de carbone ou bien un résidu acyle COR⁵ avec 1-6 atomes de carbone.

6. Composés selon les revendications 1-3,
caractérisés en ce que
R³ représente un résidu -(CH₂)ₒC≡CR⁷,
où o = 0, 1 ou 2 et R⁷ est un résidu alkyle ou acyle avec à chaque fois 1-6 atomes de carbone.

7. Composés selon la revendication 1,
c'est-à-dire
4-[9α,10α-époxy-17β-méthoxy-17α-(méthoxyméthyl)-3-oxoestr-4-èn-11β-yl]-benzaldéhyd-1-(E)-oxime,
4-[9α, 10α-époxy-17β-méthoxy-17α-(méthoxyméthyl )-3-oxoestr-4-èn-11β-yl]-benzaldéhyd-1-(Z)-oxime,
4-[9α,10α-époxy-17β-hydroxy-17α-(méthoxyméthyl)-3-oxoestr-4-èn-11β-yl]-benzaldéhyd-1-(E)-oxime,
4-[9α, 10α-époxy-17β-hydroxy-17α-(méthoxyméthyl )-3-oxoestr-4-èn-11β-yl]-benzaldéhyd-1-(Z)-oxime,
4-[9α,10α-époxy-17β-méthoxy-17α-(méthoxyméthyl)-3-oxoestr-4-èn-11β-yl]-benzaldéhyd-1-(E)-(O-acétyl)-oxime,
4-[9α,10α-époxy-17β-hydroxy-17α-(méthoxyméthyl)-3-oxoestr-4-èn-11β-yl]-benzaldéhyd-1-(E)-(O-acétyl)-oxime,
4-[9α,10α-époxy-17β-méthoxy-17α-(méthoxyméthyl)-3-oxoestr-4-èn-11β-yl]-benzaldéhyd-1-(E)-[O-(méthoxy)-carbonyl]-oxime,
4-[9α,10α-époxy-17β-hydroxy-17α-(méthoxyméthyl)-3-oxoestr4-èn-11β-yl]-benzaldéhyd-1-(E)-[O-(méthoxy)carbonyl]-oxime,
4-[9α,10α-époxy-17β-méthoxy-17α-(méthoxyméthyl)-3-oxoestr-4-èn-11β-yl]-benzaldéhyd-1-(E)-[O-(éthoxy)-carbonyl]-oxime,
4-[9α,10α-époxy-17β-hydroxy-17α-(méthoxyméthyl)-3-oxoestr-4-èn-11β-yl]-benzaldéhyd-1-(E)-[O-(éthoxy)-carbonyl]-oxime,
4-[9α,10α-époxy-17β-méthoxy-17α-(éthoxyméthyl)-3-oxoestr-4-èn-11β-yl]-benzaldéhyd-1-(E)-oxime,
4-[9α,10α-époxy-17β-hydroxy-17α-(éthoxyméthyl )-3-oxoestr-4-èn-11β-yl]-benzaldéhyd-1-(E)-oxime,
4-[9α,10α-époxy-17β-éthoxy-17α-(méthoxyméthyl )-3-oxoestr-4-èn-11β-yl]-benzaldéhyd-1-(E)-oxime,
4-[9α,10α-époxy-17β-méthoxy-17α-(2-propoxyméthyl )-3-oxoestr-4-èn-11β-yl]-benzaldéhyd-1-(E)-oxime,
4-[9α,10α-époxy-17β-hydroxy-17α-(2-propoxyméthyl)-3-oxoestr-4-èn-11β-yl]-benzaldéhyd-1-(E)-oxime,
4-[9α,10α-époxy-17β-méthoxy-17α-(méthoxyméthyl)-3-oxoestr-4-èn-11β-yl]-benzaldéhyd-1-(E)-[O-phenyloxy)-carbonyl]-oxime,
4-[9α,10α-époxy-17β-hydroxy-17α-(méthoxyméthyl)-3-oxoestr-4-èn-11β-yl]-benzaldéhyd-1-(E)-[O-(N-éthylamino)-carbonyl]-oxime,
4-[9α,10α-époxy-17β-méthoxy-17α-(méthoxyméthyl)-3-oxoestr-4-èn-11β-yl]-benzaldéhyd-1-(E)-[O-(N-éthylamino)-carbonyl]-oxime,
4-(17α-chlorométhyl-9α,10α-époxy-17β-hydroxy-3-oxoestr-4-èn-11β-yl]-benzaldéhyd-1-(E)-oxime,
4-[9α,10α-époxy-17β-acétoxy-17α-(méthoxyméthyl)-3oxoestr-4-èn-11β-yl]-benzaldéhyd-1-(E)-oxime,

8. Procédé pour la production des composés selon la revendication 1 et leurs sels pharmaceutiquement acceptables,
caractérisé en ce qu'on fait réagir des composés de la formule générale II 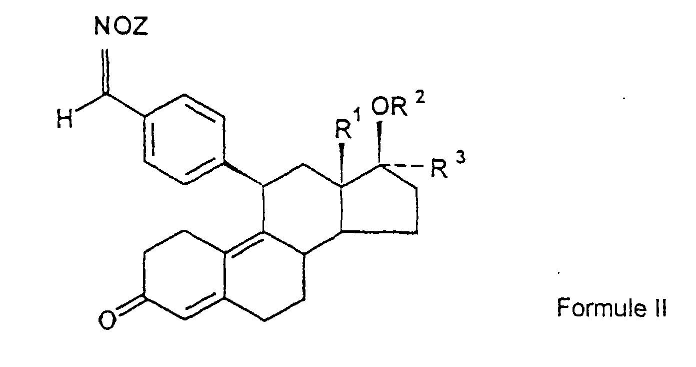 où
R¹ est un atome d'hydrogène ou un résidu alkyle avec 1-6 atomes de carbone,
R² indique un atome d'hydrogène, un groupe alkyle, aryle, aralkyle ou alkylaryle avec à chaque fois 1-10 atomes de carbone, un résidu acyle avec 1-10 atomes de carbone ou un résidu -CONHR⁴ ou COOR⁴,
où R⁴ signifie un atome d'hydrogène, un résidu alkyle, aryle, aralkyle ou alkylaryle avec à chaque fois 1-10 atomes de carbone,
R³ indique un atome d'hydrogène, un groupe alkyle, aryle, aralkyle ou alkylaryle avec à chaque fois 1-10 atomes de carbone,
un résidu -(CH₂)ₙCH₂Y,
où n = 0, 1 ou 2, Y indique un atome de fluor, de chlore, de brome ou d'iode, un groupe cyano, azido ou rhodano, un résidu OR⁵ ou SR⁵,
où R⁵ est un atome d'hydrogène, un résidu alkyle, aryle, aralkyle ou alkylaryle avec à chaque fois 1-10 atomes de carbone ou bien un résidu acyle COR⁵ avec 1-10 atomes de carbone,
un résidu OR⁵,
où R⁵ est un atome d'hydrogène, un résidu alkyle, aryle, aralkyle ou alkylaryle avec à chaque fois 1-10 atomes de carbone ou un résidu acyle COR⁵ avec 1-10 atomes de carbone,
un résidu -(CH₂)ₘ-CH=CH(CH₂)ₙ-R⁶,
où m = 0, 1, 2 ou 3 et n = 0, 1 ou 2 et
R⁶ indique un atome d'hydrogène, un résidu alkyle, aryle, aralkyle ou alkylaryle à chaque fois ayant 1 à 10 atomes de carbone ou bien un groupe hydroxyle, un groupe alcoxy ou acyloxy avec à chaque fois 1-10 atomes de carbone,
un résidu -(CH2)ₒC≡CR⁷,
où o = 0, 1 ou 2 et R⁷ indique un atome d'hydrogène, un atome de fluor, de chlore, de brome ou d'iode, un résidu alkyle, aryle, aralkyle ou alkylaryle avec à chaque fois 1-10 atomes de carbone ou un résidu acyle avec 1-10 atomes de carbone
Z indique un atome d'hydrogène, un groupe alkyle, aryle, alkylaryle ou arylalkyle avec à chaque fois 1-10 atomes de carbone, un résidu acyle avec 1-10 atomes de carbone,
un résidu COHNR⁴ ou COOR⁴,
où R⁴ signifie un atome d'hydrogène, un résidu alkyle, aryle, aralkyle ou alkylaryle avec 1-10 atomes de carbone,
ou bien indique un atome d'un métal alcalin ou un métal alcalino-terreux,
ainsi que leurs sels pharmaceutiquement acceptables,
avec un agent d'oxydation comme des péracides ou du péroxyde d'hydrogène dans des solutions ou des suspensions,
le groupe 11β-benzaldimine se trouvant, le cas échéant, libre est estérifié, étérifié ou est transformé en un autre composé de la formule I ou bien en un sel acceptable.
